# EUROPEAN PATENT APPLICATION

(11) **EP 4 825 449 A1**
(43) Date of publication of application: **21.10.2026**
(21) Application number: 25382388.4
(22) Date of filing: 15.04.2025
(51) Int. Cl.: A61P 37/04, C07K 16/44

(54) **ANTIBODIES AGAINST THE LINEAR FORMS OF STAPHYLOCOCCUS AUREUS AUTOINDUCING PEPTIDES**

(71) Applicant: Consejo Superior De Investigaciones Científicas, 28006 Madrid (ES); Consorcio Centro de Investigación Biomédica en Red, 28029 Madrid (ES)
(72) Inventor: CASTRO AGIRRE, Nerea, E-08034 Barcelona (ES); FERRERO RUEDAS, Carla, E-08034 Barcelona (ES); PASCUAL DURAN, Nuria, E-08034 Barcelona (ES); ROYO EXPÓSITO, Miriam, E-08034 Barcelona (ES); MARCO COLÁS, M. Pilar, E-08034 Barcelona (ES); ACOSTA CRESPO, Gerardo Alexis, E-28029 Madrid (ES); SALVADOR VICO, Juan Pablo, E-28029 Madrid (ES)
(74) Representative: Isern Patentes y Marcas S.L.

(57) **Abstract**

The invention relates to a hapten of formula (A) and to a conjugate comprising at least a hapten according to the invention in combination with a second component selected from the group consisting of an antigenicity carrier protein or a fragment thereof, a detectable labelling agent, an oligonucleotide, a polysaccharide, a polymer and a support. In addition, the invention also relates to methods for diagnosing a *S*. *aureus* infection, for detecting and/or quantifying linear autoinducing peptides from *S*. *aureus* in a sample and method for the identification of *S*. *aureus* in a sample which requires using the antibody of the invention.

## Description

### FIELD OF THE INVENTION

The present invention relates to the field of antibodies and their use in diagnostic methods.

### BACKGROUND OF THE INVENTION

S*taphylococcus aureus* is a gram-positive bacterial human pathogen that causes a wide variety of clinical diseases'. This bacterium belongs to the group of so-called ESKAPE pathogens, a classification of multidrug resistant "superbugs" based on prevalence, more than 10-years trend of resistance, transmissibility, treatability and preventability in hospital and community settings. This group of microorganisms represent a serious global health threat for which traditional therapeutic options have become limited.

*S*. *aureus* is found in the environment and normal human flora (mainly in skin and mucus membranes), where the bacteria can naturally reside on the human body without causing infection ². When *S*. *aureus* resides in mucus membranes, it is most frequently colonizing the nasal area'. However, it has the ability to cause skin and soft-tissue infections, as well as bone and joint infections, which can sometimes turn rapidly into a bloodstream infection^{3, 4}. In fact, *S*. *aureus* is one of the leading causatives of bacteremia, entailing mortality rates of 65-70% in the pre-antibiotic era, and 20-40% mortality after 30 days of giving appropriate treatment ⁵.

The epidemiology of *S*. *aureus* infections involves both healthcare associated infections (HAls) and community-associated skin and soft tissue infections (CAls). HAIs are particularly seen in infective endocarditis (IE) and prosthetic device infections ^{3, 4, 6}, where *S*. *aureus* became the most prevalent species of acute infections among patients. CAIs caused by *S*. *aureus* usually involve the release of virulence factors and resistance to β-lactam antibiotics, such as methicillin, nafcillin, oxacillin, and cephalosporins ^{7, 8}. Endovascular infections, pneumonia, septic arthritis, endocarditis, and bacteraemia are the main pathological signs manifested in these cases ⁹. The evolution of antimicrobial resistance in *S*. *aureus* is mainly driven by horizontal gene transfer of antimicrobial resistance genes, although chromosomal mutation has also proved to be essential for novel resistance determinants ¹⁰. Methicillin-resistant *S*. *aureus* (MRSA) constitutes a major public health threat, which has caused infections in epidemic waves ¹¹. MRSA strains contribute to the high incidence of lower respiratory tract infections , the world's most deadly communicable disease, ranked as the 4^{th} leading cause of death ¹². Therefore, there is an increasing urgent need of novel strategies to deal with this pathogen. From one hand, the correct detection and fast identification of *S*. *aureus* is crucial for an adequate treatment and prevention of exacerbation. On the other hand, the lack of diagnostic methods capable of providing reliable and fast results has led medical personnel to prescribe and misuse broad-spectrum antibiotics, contributing to the antimicrobial resistance.

Traditional diagnostic approaches to detect infections often fall short in providing early detection. Current methods are based on the culture techniques, which can take up to 72h in order to obtain conclusive results, commonly due to the low of the technique itself. Moreover, culturing may not lead to the specific identification of the causative bacterium in the presence of closely related species with similar phenotype. Molecular detection tools have emerged as an interesting approach to overcome the lack of sensitivity of actual methods, such as quantitative real-time PCR (qPCR) and MALDI-TOF-MS, allowing the detection of different bacteria in either isolates or clinical samples. However, these methods still require tedious extraction and/or purification steps, expensive equipment, and highly qualified personnel. All this causes an unaffordable delay in treatment administration, increasing the need of more rapid and reliable diagnostic tools. Immunochemical techniques seem like a promising alternative and offer a great adaptability when used in electrochemical and optical sensors.

The quorum sensing (QS) is a population-dependent bacteria communication system that controls the genetic expression of virulence factors and crucial survival mechanisms during pathogenesis. This communication process relies on the release of low molecular weight signalling molecules to the extracellular matrix, autoinducing peptides, whose concentration will increase as a function of population density. These autoinducing peptides (AIPs 1-4) are 7 to 9 cyclic thiodepsipeptides (C-terminal carboxylic group forms a thiolactone with the thiol of a Cys) produced and matured inside the bacterium S. *aureus* and secreted as the functionally active form to the extracellular media. These cyclic peptides are detected by the surrounding *Staphylococcus aureus* membrane receptors (AgrC) in the cyclic form, thereby activating the quorum sensing (Tal-Gan et al., 2013; Journal of the American Chemical Society). There have been reported clear associations between the agr type and virulence, the ability to form biofilms, and the generation of resistance (AMR).

There have been reported several immunoassays for detection of autoinducing peptides (AIPs) of *S*. *aureus* (Montagut et al., ACS Infect. Dis. 2022, 8, 645) and Janda et al (WO2009055054) as specific biomarkers of infection. According to these results, preservation of thiolactone functionality was found to be crucial to achieve detectability in the low nanomolar range.

Therefore, there is a need for developing novel diagnostic methodologies, particularly based on immunochemistry, able to substitute those described in the state of the art for the detection of infections caused by *Staphylococcus aureus* in biological samples.

### BRIEF DESCRIPTION OF THE INVENTION

The inventors have demonstrated that the linear form of AIPs is unexpectedly present in the extracellular medium of bacterial cultures, not earlier reported in the literature, leading to hypothesise that the thiolactone bond undergoes hydrolysis in the extracellular medium, thereby opening the cyclic peptide.

The total quantity of AIPs in a sample has never been measurable because it was unknown that AIPs exist also in the open (linear) form; thus, no attempt to detect them has been made. Consequently, the total quantity of AIPs released by the bacterium has never been measured.

Therefore, the present invention is directed towards solving the problem of detecting infections caused by *S*. *aureus* by providing for the first time an immunochemical assay for the identification and/or quantification of molecules from agr quorum sensing system of *S*. *aureus,* allowing their evaluation as biomarkers of infection for diagnostic purposes.

In a first aspect, the invention relates to a hapten of formula (A) 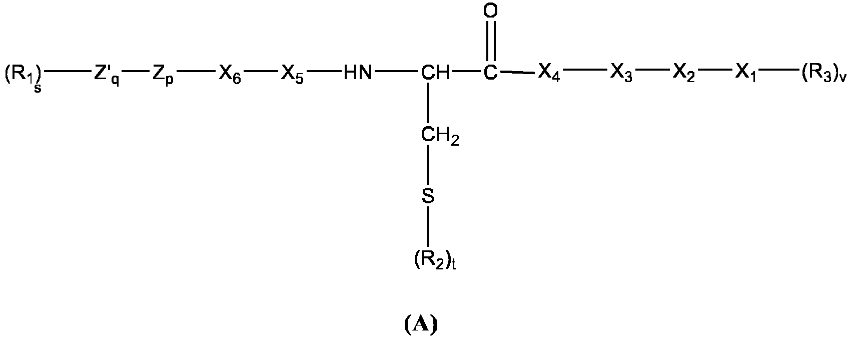 wherein
X₁ is selected from the group consisting of Methionine (M), Phenylalanine (F) and Leucine (L); X₂ is selected from the group consisting of Isoleucine (I) and Leucine (L); X₃ is selected from the group consisting of Phenylalanine (F) and Serine (S); X₄ is selected from the group consisting of Aspartic acid (D), Serine (S) and Tyrosine (Y); X₅ is selected from the group consisting of Threonine (T), Alanine (A) and Asparagine (N); X₆ is selected from the group consisting of Serine (S), Asparagine (N) and Isoleucine (I);
Z is selected from the group consisting of Tyrosine (Y) and Valine (V) and Z' is Glycine (G);
p, q are, independently, selected between 0 and 1
R₁, R₂ and R₃ are, independently, a spacer arm selected from the group consisting of R₄ and COR₄;
R₄ is selected from the group consisting of -(CH₂CH₂O)ₙ-, (C2-C10) alkyl, (C3-C10) alkenyl, (C3-C10) alkynyl and -(CH₂)m-aryl-(CH₂)r, substituted by -SH, NH₂, -OH, - COOH, -CHO, -C≡CH, halogen or -N_{3;} where n is an integer number selected between 2 and 5, m and r are selected, independently, between 0 and 3;
s, t and v are independently selected between 0 and 1; where if s is 1, t and v are 0; if t is 1, s and v are 0; and if v is 1, s and t are 0.

In a second aspect, the invention relates to a conjugate comprising at least a hapten according to the invention in combination with a second component selected from the group consisting of an antigenicity carrier protein or a fragment thereof, a detectable labelling agent, an oligonucleotide, a polysaccharide, a polymer and a support.

In a third aspect, the invention relates to the use of the conjugate according to the invention for obtaining an antibody or antisera.

In a fourth aspect, the invention relates to an antibody or antisera specific for a linear peptide selected from the group consisting of SEQ ID NO: 1, 2, 3 and 4.

In a fifth aspect, the invention relates to an *in vitro* method for detecting and/or quantifying linear autoinducing peptides from *S*. *aureus* in a sample which comprises the use of an antibody or antiserum according to the invention.

In a sixth aspect, the invention relates to an *in vitro* method for diagnosing a *S*. *aureus* infection which comprises hydrolyzing a sample obtained from a patient in suitable conditions, and determining the amount of linear autoinducing peptides using an antibody according to the invention.

In a seventh aspect, the invention relates to an *in vitro* method for the identification of *S*. *aureus* in a sample which comprises using an antibody according to the invention.

In an eight aspect, the invention relates to a kit for detecting and/or quantifying linear autoinducing peptides from *S*. *aureus* in a sample characterized in that it comprises at least one antibody specific for a linear peptide, wherein the antibody is the antibody according to the invention, or an antiserum comprising said antibody, or a conjugate according to any the invention, or any combination thereof.

### DESCRIPTION OF THE FIGURES

Figure 1. Calibration curves of the As429/AIP3c-BSA ELISA for the detection of AIP3o in buffer (PBST 10 mM) and TSB diluted 1/2 with PBST under different conditions of BSA conjugate and As dilution (see Table 11 for additional information). Each calibration point was measured in duplicates.

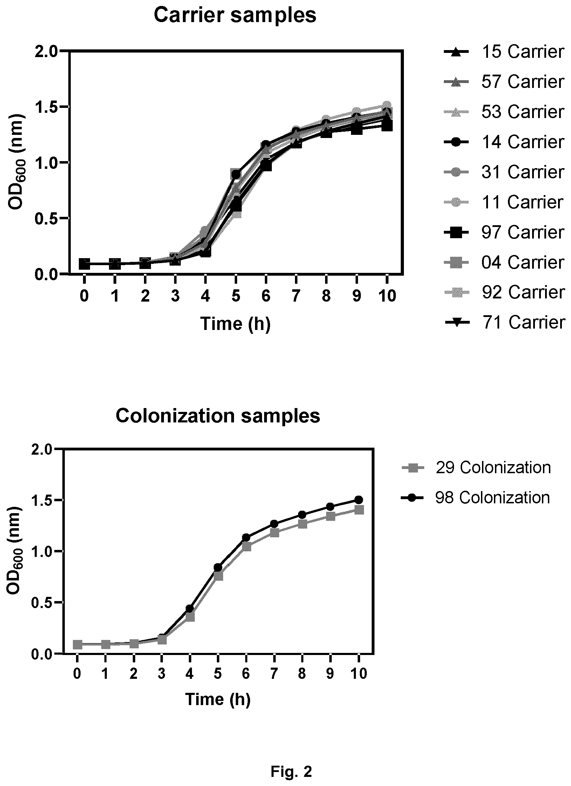

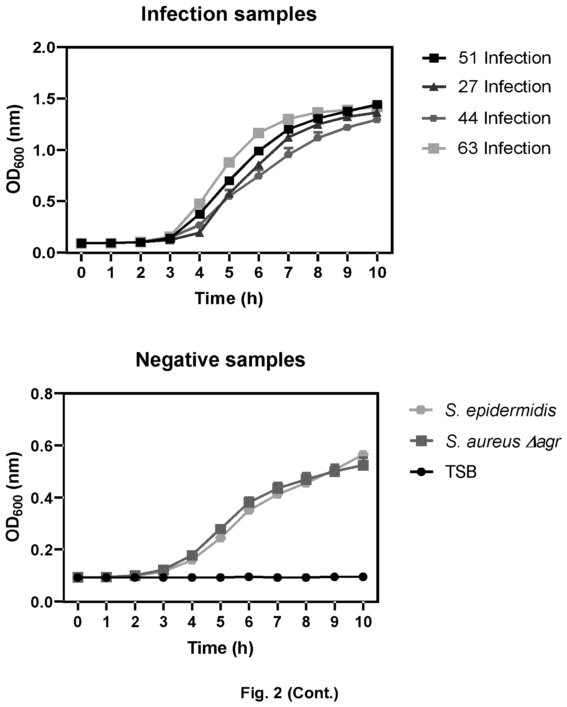

Figure 2. Growth curves of agr-III type *S*. *aureus* clinical isolates from patients of the Institute Germans Trias I Pujol (IGTP). The readout was performed at an OD600 at the same exact conditions inside the Varioskan^{™} LUX microplate reader, at 37°C and 180 rpm shaking speed.
Figure 3. Linear AIP3 (AIP3o) levels in clinical *S*. *aureus* isolates grown in TSB broth at the same exact conditions inside the Varioskan^{™} LUX microplate reader, at 37°C and 180 rpm. The AIP3o measurements were performed with the ELISA optimized with the As429FB obtained from a rabbit immunized with linear AIP3 hapten.
Figure 4. Calibration curves of the As434/AIP2c-BSA ELISA for the detection of AIP2o in buffer (PBST 10 mM) and TSB diluted 1/2 with PBST under different conditions of BSA conjugate and As dilution (see Table 8 for additional information). Each calibration point was measured in duplicates.

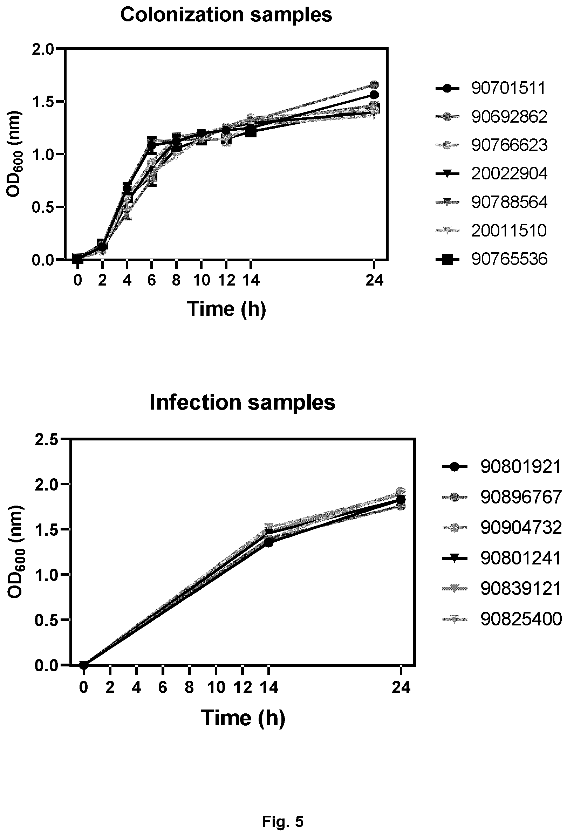

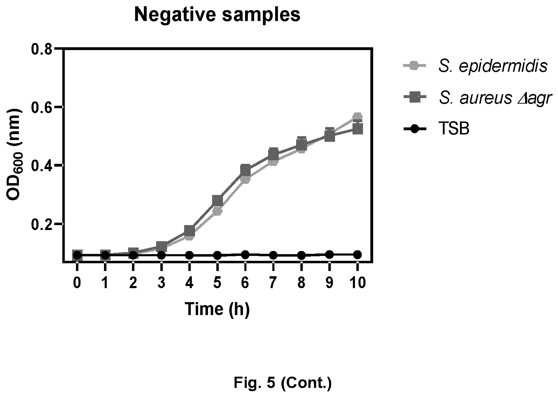

Figure 5. Growth curves of agr-II type *S*. *aureus* clinical isolates from patients of the Institute Germans Trias I Pujol (IGTP). The readout was performed at an OD600 with a Multiskan SkyHigh Microplate Spectrophotometer at times 0, 14 and 24 hours of growth at 37°C and 180 rpm shaking speed.
Figure 6. AIP2 total levels in clinical *agr*-II S. aureus isolates grown in TSB broth, at 37°C and 180 rpm shaking speed. The AIP2o measurements, after hydrolysis of the sample, were performed with the ELISA optimized with pAb 434FB. Grey bars correspond to the closed fraction of the AIP2 molecule (AIP2c), and black bars correspond to the linear fraction of AIP2 (AIP2o).

### DETAILED DESCRIPTION OF THE INVENTION

The present invention provides an immunochemical assay for the identification and/or quantification of molecules from *agr* quorum sensing system of *Staphylococcus aureus,* allowing their evaluation as biomarkers of infection (or disease) for diagnostic purposes.

### Hapten of the invention

In a first aspect, the invention relates to a hapten of formula (A) 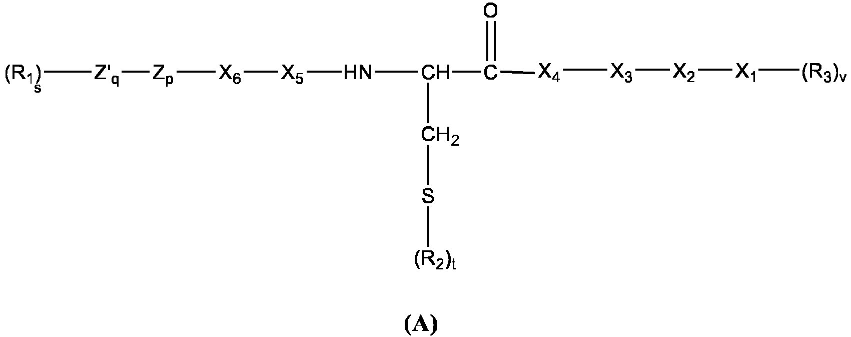 wherein
X₁ is selected from the group consisting of Methionine (M), Phenylalanine (F) and Leucine (L); X₂ is selected from the group consisting of Isoleucine (I) and Leucine (L); X₃ is selected from the group consisting of Phenylalanine (F) and Serine (S); X₄ is selected from the group consisting of Aspartic acid (D), Serine (S) and Tyrosine (Y); X₅ is selected from the group consisting of Threonine (T), Alanine (A) and Asparagine (N); X₆ is selected from the group consisting of Serine (S), Asparagine (N) and Isoleucine (I);
Z is selected from the group consisting of Tyrosine (Y) and Valine (V) and Z' is Glycine (G);
p, q are, independently, selected between 0 and 1
R₁, R₂ and R₃ are, independently, a spacer arm selected from the group consisting of R₄ and COR₄;
R₄ is selected from the group consisting of -(CH₂CH₂O)ₙ-, (C2-C10) alkyl, (C3-C10) alkenyl, (C3-C10) alkynyl, and -(CH₂)m-aryl-(CH₂)r, substituted by -SH, NH₂, -OH, - COOH, -CHO, -C≡CH, halogen or -N_{3;} where n is an integer number selected between 2 and 5, m and r are selected, independently, between 0 and 3;
s, t and v are independently selected between 0 and 1; where if s is 1, t and v are 0; if t is 1, s and v are 0; and if v is 1, s and t are 0.

"Hapten", as used herein relates to a molecule, which by itself is not capable of generating an immune response in an animal and needs to be bound to a carrier molecule to generate an immune response. Therefore, the hapten is a peptide analog of formula A of non-immunogenic character with the capacity of inducing the formation of antibodies when it is bound to a carrier molecule, in particular a carrier protein. The haptens of the invention are structurally related to AIP1, AIP2, AIP3 and AIP4 secreted by the Gram-positive bacterium S. *aureus.*

In a preferred embodiment, X₁ is M, X₂ is I, X₃ is F, X₄ is D, X₅ is T, X₆ is S, Z is Y and q is 0 in Z_{q}' in the hapten of formula (A).

In another preferred embodiment, X₁ is F, X₂ is L, X₃ is S, X₄ is S, X₅ is A, X₆ is N, Z is V and Z' is G in the hapten of formula (A).

In another preferred embodiment, X₁ is L, X₂ is L, X₃ is F, X₄ is D, X₅ is N, X₆ is I; and p and q are 0 in Zₚ and Z_{q}' in the hapten of formula (A).

In another preferred embodiment, X₁ is M, X₂ is I, _{X3} is F, X₄ is Y, X₅ is T, X₆ is S, Z is Y and q is 0 in Z_{q}' in the hapten of formula (A).

Haptens mimicking the native structure of linear AlPs can be synthesized by using any method known in the art such as chemical processes of organic synthesis, including a functionalized spacer arm susceptible of being conjugated to a carrier protein. In order to maximize the exposure of the most important epitopes, in a particular embodiment of the present invention, the spacer arm was placed at the amino groups (-NH₂) of the N-terminal amino acid residue of the linear peptidic structure, particularly the residues Tyrosine (Y), Glycine (G) and Isoleucine (I). In another preferred embodiment, the spacer arm is placed at the C-terminus of the linear peptidic structure. In another preferred embodiment, the spacer is placed at the S-terminus of the linear peptidic structure.

"Spacer arm", or linker, in a hapten, as used herein, relates to a component suitable for the covalent attachment of the hapten to other components such as a carrier protein. These molecule functions to extend the distance of the hapten from the surface of the second component of the conjugate such as a carrier.

In a preferred embodiment, the length of the spacer arm is equivalent to the length of three methylene units, up to 10 methylene units. In a more preferred embodiment, the length of the spacer arm is equivalent to the length of between 3 and 10 methylene units. In another more preferred embodiment, the length of the spacer arm is equivalent to the length of four methylene units, the length of the spacer arm is equivalent to the length of five methylene units, the length of the spacer arm is equivalent to the length of six methylene units, the length of the spacer arm is equivalent to the length of seven methylene units, the length of the spacer arm is equivalent to the length of eight methylene units, the length of the spacer arm is equivalent to the length of nine methylene units or the length of the spacer arm is equivalent to the length of ten methylene units, The length of the spacer arm can be determined by any method known by the skilled person in the art.

In a preferred embodiment, the spacer arm comprises hydrophilic groups.

"Hydrophylic group" as used herein, relates to propensity of certain groups to be water-soluble and interact favourably with water molecules. These groups are typically polar or charged, allowing them to form hydrogen bonds with water, which enhances their solubility in aqueous environments. The hydrophilic PEG groups increase reagent and conjugate solubility, minimize toxic and immunological effects compared to non-PEG spacers, and provide bioconjugation without steric hindrance.

In another preferred embodiment, the spacer arm comprises alkyl groups.

In another preferred embodiment, the spacer arm comprises alkenyl groups.

In another preferred embodiment, the spacer arm comprises aryl groups.

In another preferred embodiment, the spacer arm comprises alkynyl groups.

"Alkyl group", as used herein relates to refers to a straight or branched hydrocarbon chain radical consisting of carbon and hydrogen atoms, containing no unsaturation, and which is attached to the rest of the molecule by a single bond. Alkyl groups preferably have from 2 to about 10 carbon atoms. One more preferred class of alkyl groups has from 2 to about 12 carbon atoms; and even more preferably from 1 to about 8 carbon atoms. Alkyl groups having 1, 2, 3, 4, 5, 6, 7 or 8 carbon atoms are particularly preferred. Ethyl, n-propyl, iso-propyl and butyl, pentyl, hexyl, heptyl, octyl including n-butyl, tert-butyl, sec-butyl and iso-butyl are particularly preferred alkyl groups. As used herein, the term alkyl, unless otherwise stated, refers to both cyclic and noncyclic groups, although cyclic groups will comprise at least three carbon ring members, such as cyclopropyl or cyclohexyl.

"Alkenyl group", as used herein relates to a straight or branched hydrocarbon chain radical consisting of carbon and hydrogen atoms, containing one or more unsaturated bonds, and which is attached to the rest of the molecule by a single bond. The term "C3-C10 alkenyl" refers to a linear or branched hydrocarbon chain radical consisting of carbon and hydrogen atoms, containing one or more unsaturated bonds, having between C3-C10 alkenyl") carbon atoms and which is attached to the rest of the molecule by a single bond. Examples of alkenyl groups include propenyl, allyl, butenyl, 1-methyl-2-buten-1-yl, and the like.

"Aryl group" as used herein relates to single and multiple ring groups, including multiple ring compounds that contain separate and/or fused aryl groups. Typical aryl groups contain from 1 to 3 separated and/or fused rings and from 6 to about 18 carbon ring atoms. In a particular embodiment the aryl group is a fused bicyclic aromatic ring wherein two aromatic rings are fused. Preferably aryl groups contain from 5 to about 10 carbon ring atoms. Specially preferred aryl groups include phenyl, benzyl, naphthyl, biphenyl, phenanthryl, anthracyl and the like. The term includes but is not limited to, phenyl, naphthyl, tetrahydronaphthyl, indanyl, indenyl, and the like. In a preferred embodiment the aryl is phenyl.

"Alkynil group" as used herein relates to a straight or branched hydrocarbon chain radical containing at least three carbon atoms and at least one carbon-carbon triple bond, and which is attached to the rest of the molecule by a single bond. Alkynyl radicals according to the invention have from 3 to about 10, 3 to about 8 or 3 to about 6 carbon atoms. In a particular embodiment, the alkynyl group is propynyl (e.g. 1-propynyl, 2-propynyl), or butynyl (e.g. 1-butynyl, 2-butynyl, 3-butynyl).

According to the invention, the spacer arm can be bound at any one of R₁, R₂ or R₃
R₁, R₂ and R₃ and are, independently, a spacer arm selected from the group consisting of R₄ and COR₄;
R₄ is selected from the group consisting of -(CH₂CH₂O)ₙ-, (C2-C10) alkyl, (C3-C10) alkenyl, (C3-C10) alkynyl and -(CH₂)m-aryl-(CH₂)r, substituted by -SH, NH₂, -OH, - COOH, -CHO, -C≡CH, halogen or -N_{3;} where n is an integer number selected between 2 and 5, m and r are selected, independently, between 0 and 3; s, t and v are independently selected between 0 and 1; where if s is 1, t and v are 0; if t is 1, s and v are 0; and if v is 1, s and t are 0.

In a preferred embodiment, in order to maximize the exposure of the most important epitopes, in the present invention the spacer arm was placed at the amino groups (-NH2) of the N-terminal amino acid residue of the linear peptidic structure. In a preferred embodiment, the spacer arm is placed at the amino group of Y in the particular hapten of formula (A) wherein X₁ is M, X₂ is I, X₃ is F, X₄ is D, X5 is T, X₆ is S, Z is Y and q is 0 in Z_{q}'.

In another preferred embodiment, the spacer arm is placed at the amino group of G in the particular hapten of formula (A) wherein X₁ is F, X₂ is L, X₃ is S, X₄ is S, X₅ is A, X₆ is N, Z is V and Z' is G.

In another preferred embodiment, the spacer arm is placed at the amino group of I in the particular hapten of formula (A), wherein X₁ is L, X₂ is L, X₃ is F, X₄ is D, X₅ is N, X₆ is I; and p and q are 0 in Zₚ and Z_{q}'.

In another particular embodiment, the spacer arm is placed at the amino group of Y in the particular hapten of formula (A) wherein X₁ is M , X₂ is I, X₃ is F, X₄ is Y, X₅ is T, X₆ is S, Z is Y and q is 0 in Z_{q}'.

In a preferred embodiment, R₁ of formula (A) is a spacer arm and wherein s is 1, and t and v are 0.

In another preferred embodiment, R₂ of formula (A) is a spacer arm wherein t is 1, and s and v are 0. In another preferred embodiment, R₃ of formula (A) is a spacer arm wherein v is 1 and s and t are 0.

In another preferred embodiment, the spacer arm is -CO-(CH₂CH₂O)ₙNH₂ wherein n is an integer number selected between 2 and 5.
- (CH₂CH₂O)ₙ is also known as polyethylenglycol or PEG.

In a more preferred embodiment, the spacer arm is -CO-(CH₂CH₂O)ₙ wherein n is 2. In a more preferred embodiment, the spacer arm is -CO-(CH₂CH₂O)ₙ wherein n is 3. In a more preferred embodiment, the spacer arm is -CO-(CH₂CH₂O)ₙ wherein n is 4. In a more preferred embodiment, the spacer arm is -CO-(CH₂CH₂O)ₙ wherein n is 5. In a more preferred the spacer arm is -CO-(CH₂CH₂O)₂-NH_{2.}

In a more preferred embodiment, the hapten according to the invention is

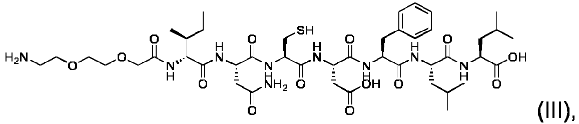

In another preferred embodiment, the hapten according to the invention is

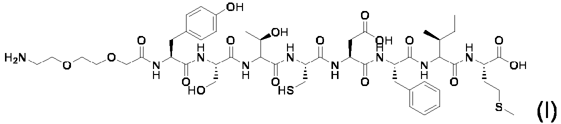

In another preferred embodiment, the hapten according to the invention is

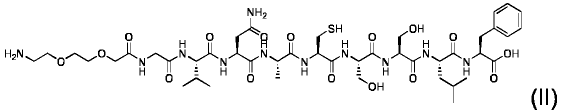

In another preferred embodiment, the hapten according to the invention is

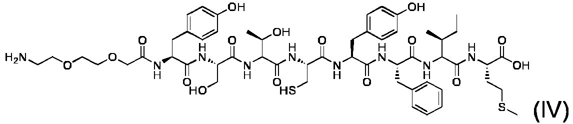

Thus, in a preferred embodiment, the hapten is NH₂-PEG(2)-CO-INCDFLL (SEQ ID NO: 3)-OH, which corresponds to hapten (III). In another preferred embodiment, the hapten is NH₂-PEG(2)-CO-YSTCDFIM (SEQ ID NO: 1)-OH, which corresponds to hapten (I). In another preferred embodiment, the hapten is NH₂-PEG(2)-CO-GVNACSSLF (SEQ ID NO: 2)-OH, which corresponds to hapten (II). In another preferred embodiment, the hapten is NH₂-PEG(2)-CO-YSTCYFIM (SEQ ID NO: 4)-OH which corresponds to hapten IV.

Throughout the description and claims, SEQ ID NO: 1 refers to YSTCDFIM, SEQ ID NO: 2 refers to GVNACSSLF, SEQ ID NO: 3 refers to INCDFLL and SEQ ID NO: 4 refers to YSTCYFIM.

### Conjugate of the invention

In another aspect, the invention relates to a conjugate comprising at least a hapten according to the invention in combination with a second component selected from the group consisting of an antigenicity carrier protein or a fragment thereof, a detectable labelling agent, an oligonucleotide, a polysaccharide, a polymer and a support. According to the invention, in case the conjugate comprises two or more haptens said haptens are identical.

In another preferred embodiment, the conjugate of the invention comprises two identical haptens in combination with one second component selected from the group consisting of an antigenicity carrier protein or a fragment thereof, a detectable labelling agent, an oligonucleotide, a polysaccharide, a polymer and a support. In another preferred embodiment, the conjugate of the invention comprises three identical haptens in combination with one second component selected from the group consisting of an antigenicity carrier protein or a fragment thereof, a detectable labelling agent, an oligonucleotide, a polysaccharide, a polymer and a support. In another preferred embodiment, the conjugate of the invention comprises four identical haptens in combination with one second component selected from the group consisting of an antigenicity carrier protein or a fragment thereof, a detectable labelling agent, an oligonucleotide, a polysaccharide, a polymer and a support. In another preferred embodiment, the conjugate of the invention comprises five identical haptens in combination with one second component selected from the group consisting of an antigenicity carrier protein or a fragment thereof, a detectable labelling agent, an oligonucleotide, a polysaccharide, a polymer and a support. In another preferred embodiment, the conjugate of the invention comprises six or more identical haptens in combination with one second component selected from the group consisting of an antigenicity carrier protein or a fragment thereof, a detectable labelling agent, an oligonucleotide, a polysaccharide, a polymer and a support.

"Conjugate" relates in the present invention to the complex formed by the covalent bond of a hapten according to the invention and a second component which is selected from the group formed by a carrier protein or a fragment thereof which gives antigenicity, a detectable tag, an oligonucleotide, a polysaccharide and a polymer or a support. Methods for producing hapten-carrier protein conjugates are known by a person skilled in the art.

In a preferred embodiment, the second component is an antigenicity carrier protein. In another preferred embodiment, the second component is a fragment of an antigenicity carrier protein. In another preferred embodiment, the second component is a detectable labelling agent. In another preferred embodiment, the second component is a polymer. In another preferred embodiment, the second component is a support. In another preferred embodiment, the second component is an oligonucleotide. In another preferred embodiment, the second component is a polysaccharide,

The term "antigenicity carrier protein" or "transport protein" or "carrier", in the context of the present invention, relates to a protein or to a fragment thereof which, on being bound to a hapten, is responsible that said hapten, in an animal organism, turns into an immunogen with the capacity of inducing antibody formation. In said conjugate the hapten is responsible for inducing the desired specificity in the immune response, and the carrier molecule is responsible for giving antigenicity to the hapten, i.e. the capacity of behaving as an antigen.

In a preferred embodiment, the antigenicity carrier protein has a molecular mass higher than 10 kDa. In a more preferred embodiment, the antigenicity carrier protein has a molecular mass higher than 15 kDa.

Examples of antigenicity carrier proteins suitable according to the invention include, without being limited to, horseshoe crab hemocyanin (HCH), keyhole limpet hemocyanin (KLH), serum albumin of various species such as bovine serum albumin (BSA), rabbit serum albumin (RSA), horseradish peroxidase (HRP), ovalbumin (OVA), conalbumin (CONA), thyroglobulin and fibrinogen, and fragments of said proteins which give antigenicity. Preferred carrier proteins according to the invention are horseshoe crab hemocyanin (HCH), bovine serum albumin (BSA) and keyhole limpet hemocyanin (KLH). Preferably the antigenicity carrier protein is selected from the group consisting of KLH and BSA. In a preferred embodiment, the antigenicity carrier protein is KLH. In another preferred embodiment, the antigenicity carrier protein is BSA.

"Detectable labelling agent" or detectable agent, as used herein refer to biocompatible compounds with capacity to be detected either directly or indirectly, the use of which facilitates the differentiation of different parts of the image, by increasing the "contrast" between those different regions of the image. They refer to an atom, molecule, compound or other substance that is useful in diagnosing, detecting or visualizing the presence of linear autoinducing peptides. According to the embodiments described herein, detectable labels may include, but are not limited to, radioactive substances (e.g., radioisotopes, radionuclides, radiolabels or radiotracers), dyes, contrast agents, fluorescent compounds or moieties, luminescent compounds or moieties, enzymes and enhancing agents (e.g., paramagnetic ions).

In a preferred embodiment, the detectable labelling agent is an enzyme. Enzymes that may be used as imaging agents in accordance with the embodiments of the disclosure include, but are not limited to, horseradish peroxidase, alkaline phosphatase, acid phosphatase, glucose oxidase, β-galactosidase, β-glucoronidase or β-lactamase. Such enzymes may be used in combination with a chromogen, a fluorogenic compound or a luminogenic compound to generate a detectable signal.

In a preferred embodiment, the detectable agent is a dye, i.e. a molecule detectable by optical means. The dye may be a fluorescent, luminescent or otherwise optically detectable dye.

Preferably, the detectable agent is a fluorescent compound or fluorophore. A "fluorophore", as described herein, can be any small molecule that can re-emit light upon light excitation. Fluorescent compounds or moieties are known in the art and will be readily apparent to one of ordinary skill. Many fluorescent compounds are commercially available with activated groups used to react with other compounds.

The detectable agent is preferably a fluorescent biocompatible dye capable of detection either directly or indirectly in an optical imaging procedure, preferably using visible or near infrared light.

In an embodiment, the fluorescent compound or moiety absorbs and emits light of wavelength 350-850 nm, more preferably 450-800 nm, and most preferably 450-750 nm.

"An oligonucleotide", refers to a single-stranded DNA or RNA molecule, preferably with up to 35, 30, 25, 20, 19, 18, 17, 16, 15, 14 or 13 bases in length (upper limit). The oligonucleotides of the invention are DNA or RNA molecules, preferably of at least 2, at least 5, at least 10, at least 12, at least 15, at least 16, at least 17, at least 18, at least 19, at least 20, at least 21, at least 22, at least 23, at least 25 nucleotide bases in length (lower limit). Ranges of base lengths can be combined in all different manners using the afore-mentioned lower and upper limits, for example at least 2 and up to 30 bases, at least 10 and up to 15 bases, at least 5 and up 15 bases or at least 15 and up to 18 bases.

" A polysaccharide", as uses herein, in is meant to include compounds composed of 10 saccharide units and up of hundreds and even thousands of monosaccharide units per molecule, which are held together by glycoside bonds and range in their molecular weights from around 5,000 and up to millions of Daltons. Examples of common polysaccharides include, but are not limited to starch, glycogen, cellulose, gum arabic, beta-glucans, agar, chitin, dextran, agar and carboxymethyl cellulose

"Polymer" or polymeric carrier, as used herein relates to a large molecule (macromolecule) composed of repeating structural units typically connected by covalent chemical bonds. Polymeric carriers, which can be natural (for example, proteins from bacteria or viruses), semi-synthetic or synthetic materials containing one or more functional groups to which a reactant moiety can be attached. As a way of example, a polymer may be a natural polymer, such as dextran, modified natural polymer such as carboxymethyl cellulose or synthetic polymers such as Ficoll, polyvinyl alcohol, and polyacrylamide. In a preferred embodiment, the polymer is polyethylenimine (PEI). In a preferred embodiment, the polymer is selected from the group consisting of liposomes, polysaccharides such as AECM-Ficoll, dextran, agar, carboxymethyl cellulose, and synthetic polypeptides such as poly-L-lysine and poly-L-glutamic acid.

The term "support", as used in the present invention, relates to any solid material whereto the components of the invention, in particular the antibodies, the haptens or the bioconjugates of the invention, are physically bound, thus being immobilized. Any of a wide variety of solid supports may be used in the immunoassays of the present invention. The suitable materials for the solid support are synthetic such as polystyrene, polyvinyl chloride, polyamide or other synthetic polymers, natural polymers such as cellulose, and derivative natural polymers such as cellulose acetate or nitrocellulose, and glass, especially glass fibres. The support may take the form of spheres, sticks, tubes and microassay or microtiter plates. Structures similar to sheets such as strips of paper, small plates and membranes are also suitable. The surface of the supports may be permeable and impermeable for aqueous solutions. Additional inorganic solid supports suitable for their use in the present invention include, but are not limited to, silicon, crystal, quartz, ceramic, metals and their oxides, silica, silicates, silicides, nitrides, amorphous silicon carbide and any other material suitable for microfabrication or microlithography. Additional organic solid supports suitable for their use in the present invention include, without limitation, polymers such as polyimide, acrylate, polymethylmetacrylate, polystyrene or nitrocellulose.

Conjugates may be prepared according to various methods known to anyone skilled in the field of organic and immunochemical synthesis, particularly, general procedures that are shown in this document. Starting materials for preparative methods are commercially available or can be prepared using the methods described in the literature.

In another preferred embodiment of the conjugate, the hapten is covalently bound to the second component though a cross-linker. In another preferred embodiment of the conjugate of the invention, the hapten is covalenty bound directly to the second component.

"Cross-linker", as used herein relates to bond or a short sequence of bonds that links one polymer chain to another, preferably links two polymer chains by a covalent or ionic bond, preferably by covalent bond.

According to the present invention, the second component, preferably an antigenicity carrier protein, is bonded to the hapten covalently by means of the amino acids accessible on their surface, preferably those amino acids with nucleophile-type side chains. The reactive amino acid of the proteins is selected from the list comprising, but without being limited to, cysteine, serine, tyrosine and lysine, being preferably lysine. In another preferred embodiment, For the protein cross-linking, the functional protein groups whereto to the cross-linking agents are targeted comprise amino groups, ε-amino groups of lysine, α-amino terminal groups, cysteine sulfhydryl groups (-SH or thiol groups), carbohydrate groups (in the case of glycoproteins) and carboxyl groups.

The processes to achieve the conjugation of haptens to other carrier molecules depend on the functional group present in the hapten molecule.

As a way of illustrative, non-limitative examples of Cross-linking agents of proteins can be succinimidyl iodoacetate (SIA), dimethyl pimelimidate (DMP), Dimethyl adipimidate (DMA), Dimethyl 3,3'-dithiobispropionimidate (DTBB), Dimethyl suberimidat (DMS) and N-succinimidyl-3-maleimidopropionate (N-SMP).

The binding of the hapten to a cross-linker can be performed by any method known in the art, for example by the methods disclosed in sections 2.1.1 and 2.1.2 of the Examples.

In a preferred embodiment, the cross-linker is DMP. In another preferred embodiment, the cross-linker is SIA. In a preferred embodiment, the cross-linking to SIA is carried out by the EDAC method. The crosslinking to SIA by the EDAC method can be carried out by the method described in the examples, see 2.1.2. In another preferred embodiment SIA binds the hapten via the thiol.

The second component of the conjugate can be bound to the hapten of formula (A) in position R₁, R₂ or R₃. In a preferred embodiment, the hapten of formula (A) is bound to the second component of the conjugate in position R1. In another preferred embodiment, the hapten of formula (A) is bound to the second component of the conjugate in position R2. In another preferred embodiment, the hapten of formula (A) is bound to the second component of the conjugate in position R3.

In another preferred embodiment of the conjugate of the invention, the hapten is

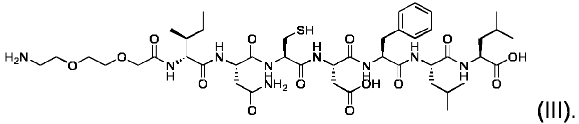

In another preferred embodiment of the conjugate of the invention, the hapten is

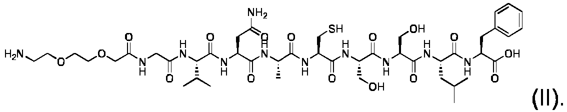

In another preferred embodiment of the conjugate of the invention, the hapten is

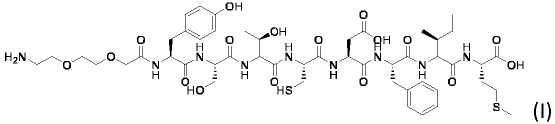

In another preferred embodiment of the conjugate of the invention, the hapten is

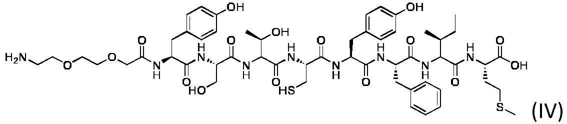

In a more preferred embodiment, the conjugate of the invention comprises the hapten (III) bound to KLH through DMP (in the examples AIP3o-DMP-KLH)
In another more preferred embodiment, the conjugate of the invention comprises the hapten (II) bound to KLH through SIA (in the examples AIP2o-SIA-KLH)
In another preferred embodiment, the conjugate of the invention comprises the hapten (I) bound to KLH through SIA. In another preferred embodiment, the conjugate of the invention comprises the hapten (IV) bound to KLH through SIA.

In a preferred embodiment the conjugate of the invention is selected from the group consisting of
BSA-X-NH-PEG(2)-CO-YSTCDFIM (SEQ ID NO: 1)-OH
KLH-X-NH-PEG(2)-CO-YSTCDFIM (SEQ ID NO: 1)-OH
BSA-X-NH-PEG(2)-CO-GVNACSSLF (SEQ ID NO: 2)-OH
KLH-X-NH-PEG(2)-CO-GVNACSSLF (SEQ ID NO: 2)-OH
BSA-X-NH-PEG(2)-CO-INCDFLL (SEQ ID NO: 3)-OH
KLH-X-NH-PEG(2)-CO- INCDFLL (SEQ ID NO: 3)-OH
BSA-X-NH-PEG(2)-CO-YSTCYFIM (SEQ ID NO: 4)-OH and
KLH-X-NH-PEG(2)-CO-YSTCYFIM (SEQ ID NO: 4)-OH, wherein X is a cross-linker agent.

In a preferred embodiment, X is DMP. In another preferred embodiment, X is SIA.

In a more preferred embodiment, the conjugate is KLH-X-NH-PEG(2)-CO-INCDFLL (SEQ ID NO: 3)-OH, wherein X is DMP.

In another preferred embodiment, the conjugate is KLH-X-NH-PEG(2)-CO-YSTCDFIM (SEQ ID NO: 1)-OH, wherein X is DMP.

In another preferred embodiment, the conjugate is KLH-X-NH-PEG(2)-CO-GVNACSSLF (SEQ ID NO: 2)-OH, wherein X is SIA.

In another preferred embodiment, the conjugate is KLH-X-NH-PEG(2)-CO-YSTCDFIM (SEQ ID NO: 1)-OH, wherein X is SIA
In another preferred embodiment, the conjugate is KLH-X-NH-PEG(2)-CO-INCDFLL (SEQ ID NO: 3)-OH, wherein X is SIA
In another preferred embodiment, the conjugate is KLH-X-NH-PEG(2)-CO-YSTCYFIM (SEQ ID NO: 4)-OH, wherein X is SIA
In another preferred embodiment, the conjugate is immobilized on a solid support.

All the terms and embodiments previously described are equally applicable to this aspect of the invention.

### Uses of the conjugate of the invention.

In another aspect, the invention relates to the use of the conjugate of the invention for obtaining an antibody or antisera.

In a preferred embodiment, the invention relates to the use of the conjugate of the invention wherein the carrier protein is KLH. In another preferred embodiment, the invention relates to the use of the conjugate of the invention wherein the cross-linker is DMP or SIA. In another preferred embodiment, the invention relates to the use of the conjugate of the invention, wherein the conjugate comprises a hapten selected from the group consisting of 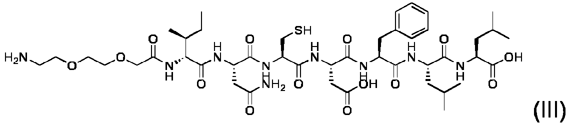 and

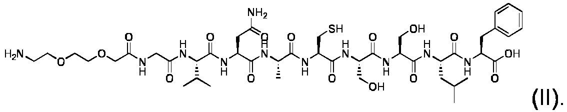

In another preferred embodiment of the use of the conjugate of the invention, the conjugate comprises a hapten selected from the group consisting of 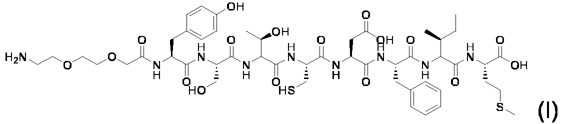 and

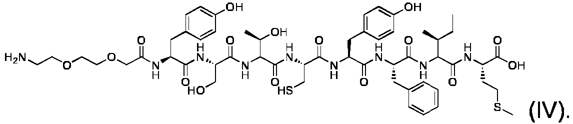

The invention also relates to a method for obtaining an antibody or antisera specific for a linear peptide selected from the group consisting of SEQ ID NO: 1, 2, 3 and 4, which comprises administering to a non-human mammal a composition comprising a conjugate according to the invention.

In a preferred embodiment the method for obtaining an antibody or antisera comprises administering a conjugate of the invention, wherein the carrier protein is KLH. In another preferred embodiment the method for obtaining an antibody or antisera comprises administering a conjugate of the invention, wherein the cross-linker is DMP or SIA. In another preferred embodiment, the method for obtaining an antibody or antisera comprises administering a conjugate of the invention, wherein the conjugate comprises a hapten selected from the group consisting of 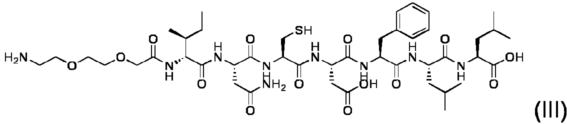 and

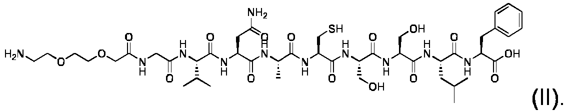

In another preferred embodiment, the method for obtaining an antibody or antisera comprises administering a conjugate of the invention, wherein the conjugate comprises a hapten selected from the group consisting of 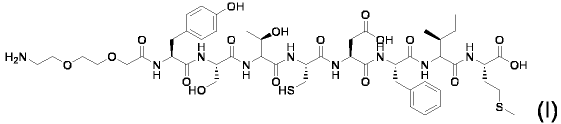 and

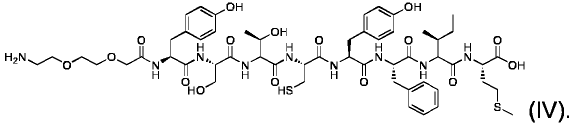

Various host animals can be immunized for injection with the conjugate according to the invention, including, but not limited to, mouse, rat, rabbit, chicken, camelid or sheep or may be a transgenic version of any of the animals mentioned above, for example a transgenic mouse with human immunoglobulin genes, which produces human antibodies after an antigenic stimulus. Other types of animals which may be immunized include mice with severe combined immunodeficiency (SCID) which have been reconstituted with human peripheral mononuclear blood cells (chimeric hu-PBMC SCID mice) or with lymphoid cells or precursors thereof, as well as mice which have been treated with a lethal total body irradiation, then protected against radiation with bone marrow cells from a mouse with severe combined immunodeficiency (SCID) and subsequently transplanted with functional human lymphocytes (the "Trimera" system). Another type of an animal to be immunized is an animal (e.g. a mouse) in whose genome an endogenous gene encoding the antigen of interest has been switched off (knocked out), for example by homologous recombination, so that, after immunization with the antigen, said animal recognizes said antigen as foreign. It is obvious to the skilled worker that the polyclonal or monoclonal antibodies produced by this method are characterized and selected by using known screening methods which include, but are not limited to, ELISA techniques.

Various adjuvants may be used to enhance the immunological response, depending on the host species, including, but not limited to, complete or incomplete Freund's adjuvant, a mineral gel such as aluminum hydroxide, surface active substances such as lysolecithin, pluronic polyol, a polyanion, a peptide, an oil emulsion, keyhole limpet hemocyanin, dinitrophenol, or an adjuvant such as BCG (bacille Calmette-Guerin) or *Corynebacterium parvum.*

For the preparation of monoclonal antibodies, any technique which provides for the production of antibody molecules by continuous cell lines in culture may be used. Hyperimmunization of an appropriate donor, generally a mouse, with the antigen is undertaken. Isolation of splenic antibody producing cells is then carried out. These cells are fused to a cell characterized by immortality, such as a myeloma cell, to provide a fused cell hybrid (Hybridoma) which can be maintained in culture and which secretes the required monoclonal antibody. The cells are then cultured in bulk and the monoclonal antibodies harvested from the culture media for use. Specific techniques include but are not limited to the hybridoma technique developed by Kohler and Milstein (1975), the human B-cell hybridoma technique (Kozbor et al., 1983) or the EBV-hybridoma technique to produce human monoclonal antibodies (Cole et al, 1985). Screening for the desired antibody can be done by techniques known in the art, such as ELISA.

For the preparation of polyclonal antibodies, the selected animal host is injected with the immunization cocktails, preferably subcutaneously. The antibody titers may be determined using an immunoassay, for example a competitive immunoassay. Thus, it may be decided toward the end of immunization whether a particular host is suitable for producing antibodies. If, for example, four immunizations are carried out, it is possible to determine the antibody titer after the third immunization and then to obtain antibodies from animals having a sufficient antibody titer. The antibodies produced are preferably obtained by taking blood from the hosts over a period of several weeks or months. Finally, the host can be bled. Serum containing the desired antibodies may be obtained from the blood obtained in a manner known per se. The whole serum thus obtained may, if required, be further purified by the skilled worker in order to concentrate the antibody fraction present therein.

Antibodies that recognize a linear peptide selected from the group consisting of SEQ ID NO: 1, 2, 3 and 4 can be identified in a simple immunoassay showing the ability of one antibody to block the binding of another antibody to a target antigen, i.e., a competitive binding assay. Competitive binding is determined in an assay in which the immunoglobulin under test inhibits specific binding of a reference antibody to a common antigen. Numerous types of competitive binding assays are known, for example: solid phase direct or indirect radioimmunoassay (RIA), solid phase direct or indirect enzyme immunoassay (EIA), sandwich competition assay (see Stahli et al., Methods in Enzymology 9:242 (1983)); solid phase direct biotin-avidin EIA (see Kirkland et al., J. Immunol. 137:3614 (1986)); solid phase direct labeled assay, solid phase direct labeled sandwich assay (see Harlow and Lane, Antibodies: A Laboratory Manual, Cold Spring Harbor Press (1988)); solid phase direct label RIA using 1-125 label (see Morel et al., Mol. Immunol. 25(1):7 (1988)); solid phase direct biotin-avidin EIA (Cheung et al., Virology 176:546 (1990)); and direct labeled RIA. (Moldenhauer et al., Scand. J. Immunol. 32:77 (1990)). Typically, such an assay involves the use of purified linear peptide selected from the group consisting of SEQ ID NO: 1, 2, 3 and 4 bound to a solid surface or cells bearing either of these, an unlabeled test immunoglobulin and a labeled reference immunoglobulin. Competitive inhibition is measured by determining the amount of label bound to the solid surface or cells in the presence of the test immunoglobulin. Usually the test immunoglobulin is present in excess. Usually, when a competing antibody is present in excess, it will inhibit specific binding of a reference antibody to a common antigen by at least 50-55%, 55-60%, 60-65%, 65-70% 70-75% or more. In another preferred embodiment, antibodies that recognize a linear peptide selected from the group consisting of SEQ ID NO: 1, 2, 3 and 4 can be identified by a ELISA. In a preferred embodiment, the method is carried out by an ELISA assay. The ELISA can be competitive or non-competitive direct ELISA, indirect ELISA or sandwich ELISA. Preferably is a competitive indirect ELISA. In another preferred embodiment, the ELISA is non-competitive indirect 2D ELISA. In case of a competitive ELISA, the corresponding closed forms of the peptides having a sequence selected from the group consisting of SEQ ID NO: 1, 2, 3 and 4 can be used.

In a preferred embodiment, the antibodies are affinity purified using methods commonly known to the skilled person. For affinity purification of polyclonal or monoclonal antibodies a suitable peptide column has to be prepared. Purified peptide or a conjugate thereof is fixed by a standard protocol to a suitable solid support as for example are Sepharose or Affi-gel, activated to covalently the antigen to the support (suitable activated solid supports are for example available from Pierce, Rockford, USA). An affinity column is then prepared from said antigen-carrying resin.

All the terms and embodiments previously described are equally applicable to this aspect of the invention.

### Antibody of the invention

In another aspect, the invention relates to an antibody or antisera obtained by the method for obtaining an antibody or antisera as defined above.

In another aspect the invention relates to an antibody or antisera specific for a linear peptide selected from the group consisting of SEQ ID NO: 1, 2, 3 and 4. In a preferred embodiment, the antibody or antisera specific for a linear peptide selected from the group consisting of SEQ ID NO: 1, 2, 3 and 4 is obtained by the method for obtaining an antibody. In a more preferred embodiment, the antibody or antisera specific for a linear peptide selected from the group consisting of SEQ ID NO: 1, 2, 3 and 4 is obtained by immunizing a non-human mammal with a conjugate according to the invention, more particularly wherein the conjugate comprises KLH as a carrier protein.

In a preferred embodiment the antibody or antisera according to the invention is specific for a linear peptide of sequence SEQ ID NO: 3. In another preferred embodiment, the antibody or antisera according to the invention is specific for a linear peptide of sequence SEQ ID NO: 2. In another preferred embodiment, the antibody or antisera according to the invention is specific for a linear peptide of sequence SEQ ID NO: 1. In another preferred embodiment, the antibody or antisera according to the invention is specific for a linear peptide of sequence SEQ ID NO: 4.

In another preferred embodiment, the antibody or antisera according to the invention specific for a linear peptide of sequence SEQ ID NO: 3 is obtained by immunizing a non-human mammal with the conjugate KLH-X-NH-PEG(2)-CO-INCDFLL (SEQ ID NO: 3)-OH, wherein X is DMP.

In another preferred embodiment, the antibody or antisera according to the invention specific for a linear peptide of sequence SEQ ID NO: 2 is obtained by immunizing a non-human mammal with the conjugate KLH-X-NH-PEG(2)-CO-GVNACSSLF (SEQ ID NO: 2)-OH, wherein X is SIA.

As used herein, an "antibody" refers to a protein consisting of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. The basic immunoglobulin (antibody) structural unit is known to comprise a tetramer or dimer. Each tetramer is composed of two identical pairs of polypeptide chains, each pair having one "light" (about 25 kD) and one 'heavy" chain (about 50-70 kD). The N-terminus of each chain defines a variable region of about 100 to 110 or more amino acids, primarily responsible for antigen recognition. The terms "variable light chain (VL)" and "variable heavy chain (VH)" refer to these variable regions of the light and heavy chains respectively. Optionally, the antibody or the immunological portion of the antibody, can be chemically conjugated to, or expressed as, a fusion protein with other proteins. In a preferred embodiment, the antibody is polyclonal. In another preferred embodiment, the antibody is monoclonal.

The term "antibody" comprises any type of known antibody such as, but not being limited to, for example, polyclonal antibodies or monoclonal antibodies, intact, or fragments thereof; and includes human, humanized and non-human origin antibodies. In the context of this invention, the term antibody relates to the immunoglobulin that the animal or a hybrid cell has synthesized specifically against the conjugated hapten of the invention. Antibodies of the invention include, but are not limited to polyclonal, monoclonal, bispecific, human, humanized or chimeric antibodies, single variable fragments (ssFv), single chain fragments (scFv), Fab fragments, F(ab') fragments, fragments produced by a Fab expression library, anti-idiotypic antibodies and epitope-binding fragments of any of the above, provided that they retain the original binding properties. Also, mini-antibodies and multivalent antibodies such as diabodies, triabodies, tetrabodies and pentabodies can be used in a method of the invention. The preparation and use of these fragments and multivalent antibodies has been described extensively in International Patent Application WO 98/29442. The immunoglobulin molecules of the invention can be of any class (i.e. IgG, IgE, IgM, IgD and IgA) or subclass of immunoglobulin molecules.

The term "specific", in the context of the antibody used in the first method of the invention, means that said antibody is capable of recognizing a particular antigen and no other different antigens.

"Antisera", as used herein, relates to blood serum containing antibodies against a specific antigen.

All the terms and embodiments previously described are equally applicable to this aspect of the invention.

### In vitro methods of the invention

In another aspect, the invention relates to an *in vitro* method for detecting and/or quantifying linear autoinducing peptides from S. *aureus* in a sample which comprises the use of an antibody or antiserum according to the invention.

The term *"in vitro",* as used herein, refers to the fact that the method is not carried out on the body of a human or animal subject, but rather on cells or fluids isolated from said subject or in a test tube.

As used herein the term "detecting" and derivations of this term refer to determining the presence, absence, amount, localization, distribution or organization of linear autoinducing peptides in a sample. According to the present invention, "detecting" also includes visualizing, determining, observing, evaluating, quantifying, measuring or imaging linear autoinducing peptides. This term includes detecting linear autoinducing peptides at a time point and detecting linear autoinducing peptides over time (also denoted herein as "monitoring" and derivations of this term). As disclosed herein, "detecting" includes determining the presence, absence, or amount of an analyte in a sample, and can include quantifying the amount of the analyte in a sample or per cell in a sample. The terms "detect" and "identify" are used interchangeably herein.

"Quantifying", as used herein, refers to determine the amount of a linear autoinducing peptide in a sample. As will be understood by those skilled in the art, the detection, although preferred to be, need not be correct for 100% of the cells to be detected or evaluated. The term, however, requires that a statistically significant portion of number of proteins can be determined. Whether a number of proteins is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90%, or at least 95%. The p-values are, preferably, 0.05, 0.01, 0.005 or lower.

In a preferred embodiment, the *in vitro* method requires detecting and/or quantifying one linear peptide. In a preferred embodiment, the method comprises determining the linear peptide AIP1o of formula 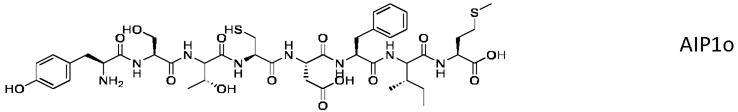 AIP1o corresponds to SEQ ID NO: 1.
In another preferred embodiment, the method comprises determining the linear peptide AIP2o of formula 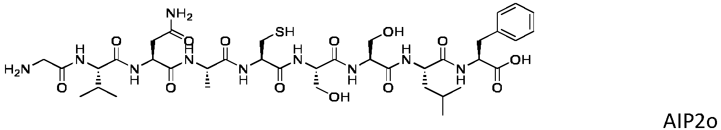 AIP2o corresponds to SEQ ID NO: 2.
In another preferred embodiment, the method comprises determining the linear peptide AIP3o of formula 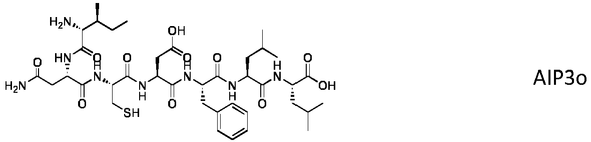 AIP3o corresponds to SEQ ID NO: 3.

In another preferred embodiment, the method comprises determining the linear peptide AIP4o of formula 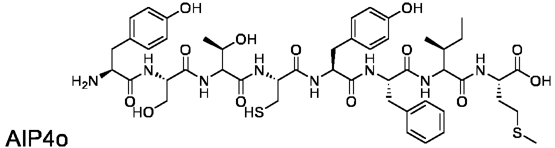 AIP4o corresponds to SEQ ID NO: 4.

The aminoacidic sequences of the linear/opened autoinducing peptides 1-4 are, respectively: H-YSTCDFIM (SEQ ID NO; 1)-OH (AIP1o), H-GVNACSSLF (SEQ ID NO 2)-OH (AIP2o), H-INCDFLL (SEQ ID NO: 3)-OH (AIP3o) and H-YSTCYFIM (SEQ ID NO: 4)-OH (AIP4o).

In another preferred embodiment, the in vitro method requires detecting and/or quantifying two linear peptides. In a more preferred embodiment, the method requires detecting and/or quantifying AIP1o and AIP2o, AIP1o and AIP3o, AIP1o and AIP4o, AIP2o and AIP3o, AIP2o and AIP4o, or AIP3o and AIP4o.

In another preferred embodiment, the in vitro method requires detecting and/or quantifying three linear peptides. In a more preferred embodiment, the method requires detecting and/or quantifying AIP1o, AIP2o and AIP3o, AIP1o, AIP2o and AIP4o, AIP1o, AIP3o and AIP4 or AIP2o, AIP3o and AIP4o.

In another preferred embodiment, the in vitro method requires detecting and/or quantifying four linear peptides. In a more preferred embodiment, the method requires detecting and/or quantifying AIP1o, AIP2o, AIP3o, AIP4o.

As the person skilled in the art will understand, the *in vitro* method for detecting and/or quantifying linear autoinducing peptides from S. *aureus* in a sample requires using an antibody or antiserum of the invention depending on the particular linear autoinducing peptide to be detected.

*"S. aureus",* as used herein relates to a gram-positive spherically shaped bacterium, a member of the Bacillota, and is a usual member of the microbiota of the body, frequently found in the upper respiratory tract and on the skin. Although *S. aureus* usually acts as a commensal of the human microbiota, it can also become an opportunistic pathogen, being a common cause of skin infections including abscesses, respiratory infections such as sinusitis, and food poisoning.

"Sample", as used herein, relates to a sample to be analyzed by the method of the invention, susceptible of containing autoinducer molecules in this linear forms (AIPo) as markers of infections caused by *Staphylococcus sp,* especially *S*. *aureus,* which has been previously obtained from the subject under study (unless indicated otherwise). Illustrative, non-limiting examples of samples include both biological samples of tissues and body fluids, such as, for example, blood, serum, plasma, saliva, sputum, ear suppurations, bronchial washes, tissue exudates, etc. In a particular embodiment, said sample is sputum. In another particular embodiment, said sample is plasma. In another particular embodiment, said sample is bronchoalveolar lavage (BAL) or bronchial aspirate (BAS). Furthermore, the sample may come, for example, from cell cultures, environmental samples such as water, soil, surface or food samples.

The detection and/or quantification linear autoinducing peptides from S. aureus in a sample which comprises the use of an antibody or antiserum according to the invention can be carry out by any method known in the art, preferably by a immunochemical technique.

The term "immunochemical technique of analysis" is an immunochemical method of analysis wherein an antibody is used which specifically binds to an antigen. The immunochemical technique of analysis is characterized by the use of specific binding properties of a particular antibody to isolate, direct and/or quantify the antigen. The immunochemical techniques comprise, without being limited to, immunoassays such as ELISA (Enzyme-Linked Immunosorbent Assay), LFIA (Lateral-flow immunoassay) Western-blot, RIA (radioimmunoassay), competitive EIA (enzyme immunoassay), DAS-ELISA (Double Antibody Sandwich-ELISA), immunocytochemical techniques and immunohistochemical techniques, techniques based on the use of biomarker, biosensor or microarray biochips, which include specific antibodies or assays based on colloidal precipitation in formats such as "dipsticks". Other immunochemical techniques include immunosensors whose transduction principle may be optical, electrochemical, piezoelectric, mass or thermometric. It also includes the immunosorbents or immunoaffinity extraction systems, which allow the selective extraction of an analyte within a complex mixture. These systems, usually of biohydrid materials, result from the stable union of the antibody to a solid support (polymer, inorganic material, metal particles, etc.), and which are used for the separation or extraction of the analyte from the rest of the matrix's components.

In a preferred embodiment, the detection and/or quantification is carried out by an immunochemical technique, preferably the immunochemical technique is an ELISA.

As a way of example, a conjugate of the invention is immobilized on a solid support acting as competitor or a coating antigen. The non-immobilized conjugate is eliminated, and the sample to be analysed together with a first antibody is added in the solid support. After an incubation of these reagents, the first antibody that has not been bound is eliminated and a second antibody conjugated with a labelling agent is added. The second antibody recognizes the first antibody during an incubation, and remaining the second antibody is eliminated. The complex may detected and/or quantified with a composition containing a chromogenic, fluorogenic and/or chemiluminescent indicator substrate. When using a chromogenic indicator substrate, the reaction is enzymatic.

In a preferred embodiment of the method for detecting and/or quantifying linear autoinducing peptides from *S*. *aureus,* the method comprises hydrolyzing the sample.

"Hydrolysing", as used herein relates to hydrolising the thiolactone bond of autoinducing peptides thereby opening the cyclic peptide and leading to the linear form of the peptides. The hydrolysis of the sample can be carried out by any method known in the art, for example by increasing the pH above 7, for example by adding NaOH, KOH, carbonate salts or hydrogenocarbonate salts.

In another aspect, the invention relates to an *in vitro* method for diagnosing a *S*. *aureus* infection which comprises hydrolyzing a sample obtained from a patient in suitable conditions, and determining the amount of linear autoinducing peptides using an antibody according to the invention.

The expression "diagnosis" or derivatives of the words, refers to the identification of the presence or characteristic of a pathological condition. It refers both to the process of attempting to determine and/or identify a possible disease in a subject, i.e. the diagnostic procedure, and to the opinion reached by this process, i.e. the diagnostic opinion. As such, it can also be regarded as an attempt at classification of an individual's condition into separate and distinct categories that allow medical decisions about treatment and prognosis to be made. As the person skilled in the art will understand, such a diagnosis may not be correct for 100% of the subjects to diagnose, although preferred it is. The term, however, requires that a statistically significant part of the subjects can be identified as suffering from a disease. The skilled in the art may determine whether the classification is statistically significant using different statistical evaluation tools well known, for example, by determination of confidence intervals, the p-value determination, Student's-test, the Mann-Whitney, etc. Preferred confidence intervals are at least, 50%, at least 60%, at least 70%, at least 80%, at least 90%) or at least 95%. The p-values are preferably, 0.05, 0.025, 0.001 or lower.

In a preferred embodiment of the method for diagnosing a *S*. *aureus* infection, the method comprises hydrolyzing the sample.

In another aspect, the invention relates to an in vitro method for the identification of S. aureus in a sample which comprises using an antibody according to the invention.

All the terms and embodiments previously described in relation to the *in vitro* method for detecting and/or quantifying linear autoinducing peptides from *S*. *aureus* are equally applicable to the method for diagnosing S. aureus infection and for the identification of S. aureus in a sample.

All the terms and embodiments previously described are equally applicable to these aspects of the invention.

### Kit of the invention.

In another aspect, the invention relates to a kit for detecting and/or quantifying linear autoinducing peptides from *S*. *aureus,* for the identification of *S*. *aureus* or for diagnosing a *S*. *aureus* infection in a sample characterized in that it comprises at least one antibody specific for a linear peptide according to any one of the invention, or an antiserum comprising said antibody, or a conjugate according to the invention, or any combination thereof.

All the terms and embodiments previously described are equally applicable to this aspect ***

### Additional aspects of the invention

1. Hapten of formula (A) 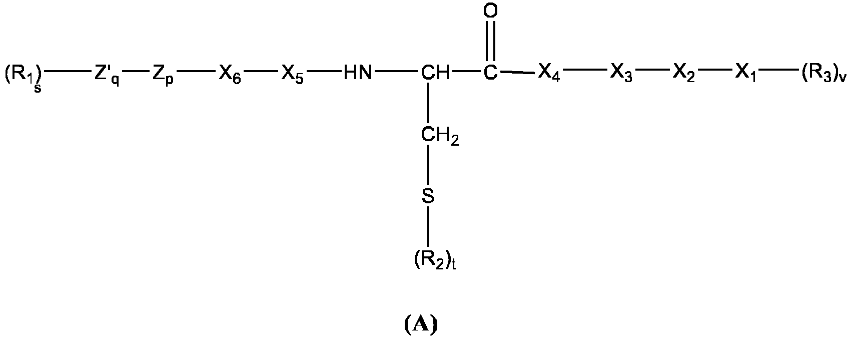 wherein
   X₁ is selected from the group consisting of Methionine (M), Phenylalanine (F) and Leucine (L); X₂ is selected from the group consisting of Isoleucine (I) and Leucine (L); X₃ is selected from the group consisting of Phenylalanine (F) and Serine (S); X₄ is selected from the group consisting of Aspartic acid (D), Serine (S) and Tyrosine (Y); X₅ is selected from the group consisting of Threonine (T), Alanine (A) and Asparagine (N); X6 is selected from the group consisting of Serine (S), Asparagine (N) and Isoleucine (I);
   Z is selected from the group consisting of Tyrosine (Y) and Valine (V) and Z' is Glycine (G);
   p, q are, independently, selected between 0 and 1
   R₁, R₂ and R₃ are, independently, a spacer arm selected from the group consisting of R₄ and COR₄;
   R4 is selected from the group consisting of -(CH₂CH₂O)ₙ-, (C2-C10) alkyl, (C3-C10) alkenyl, (C3-C10) alkynyl and -(CH₂)m-aryl-(CH₂)r, substituted by -SH, NH₂, -OH, - COOH, -CHO, -C≡CH, halogen or -N_{3;} where n is an integer number selected between 2 and 5, m and r are selected, independently, between 0 and 3;
   s, t and v are independently selected between 0 and 1; where if s is 1, t and v are 0; if t is 1, s and v are 0; and if v is 1, s and t are 0.
2- The hapten according to aspect 1 where the spacer arm is -CO-(CH₂CH₂O)₂-NH₂.
3- The hapten according to any one of aspects 1 or 2 selected from the group consisting of 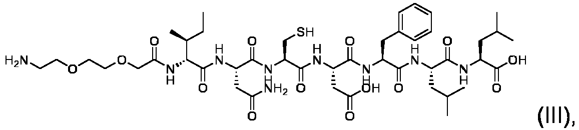 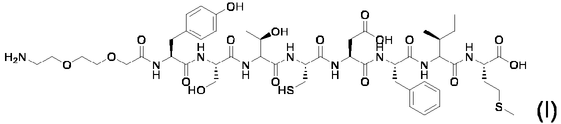 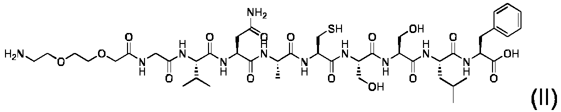 and 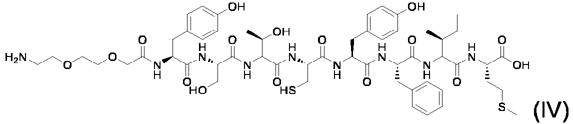
4. A conjugate comprising at least a hapten according to any one of aspects 1 to 3 in combination with a second component selected from the group consisting of an antigenicity carrier protein or a fragment thereof, a detectable labelling agent, an oligonucleotide, a polysaccharide, a polymer and a support.
5. The conjugate according to aspect 4, wherein the second component is an antigenicity carrier protein or a fragment thereof.
6- The conjugate according to aspect 5, wherein the carrier protein is selected from the group consisting of bovine serum albumin (BSA), horseshoe crab hemocyanin (HCH), keyhole limpet hemocyanin (KLH), rabbit serum albumin (RSA), horseradish peroxidase (HRP), ovoalbumin (OVA) conalbumin (CONA) thyroglobulin and fibrinogen.
7- The conjugate according to aspect 6, wherein the carrier protein is BSA or KLH.
8- The conjugate according to any one of aspects 4 to 7, wherein the hapten is bound covalently to the second component through a cross-linker.
9- The conjugate according to aspect 8, wherein the cross-linker is DMP.
10- The conjugate according to aspect 8, wherein the cross-linker is SIA *(N-*hydroxysuccinimide iodoacetate).
11-The conjugate according to any one of aspects 4 to 10, wherein the hapten is bound to the second component through the R1 position.
12. The conjugate according to any one of aspects 4 to 11 wherein the hapten is selected from the group consisting of 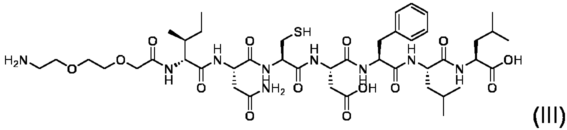 and 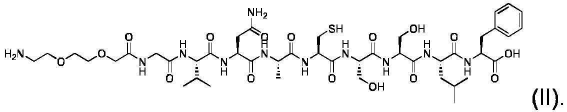
13- Use of the conjugate according to any one of aspects 4 to 12 for obtaining an antibody or antisera.
14- Use according to aspect 13, wherein the carrier protein of the conjugate is KLH and the cross-linker is DMP or SIA.
15- Use according to any one of aspects 13 or 14, wherein the conjugate comprises a hapten selected from the group consisting of 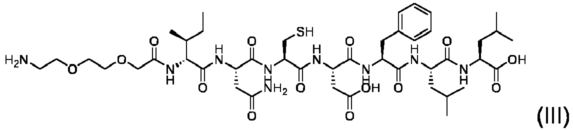 and 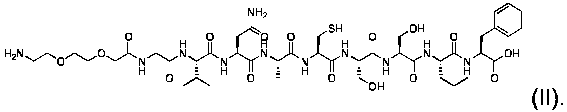
16- Antibody or antisera specific for a linear peptide selected from the group consisting of SEQ ID NO: 1, 2, 3 and 4.
17- Antibody or antisera according to aspect 16 specific for a linear peptide of sequence SEQ ID NO: 3 or SEQ ID NO: 2.
18- Antibody or antisera according to any one of aspects 16 to 17 obtained by immunizing a non-human mammal with a conjugate according to any one of aspects 4 to 12, particularly wherein the carrier protein is KLH.
19- An in vitro method for detecting and/or quantifying linear autoinducing peptides from *S*. *aureus* in a sample which comprises the use of an antibody or antiserum as defined in aspects 16 to 19.
20-The in vitro method according to aspect 19, wherein the detection and/or quantification is carried out by an immunochemical technique, preferably the immunochemical technique is an ELISA.
21- An *in vitro* method for diagnosing a *S*. *aureus* infection which comprises hydrolyzing a sample obtained from a patient in suitable conditions, and determining the amount of linear autoinducing peptides using an antibody according to any one of aspects 16 to 19.
22- An *in vitro* method for the identification of S. *aureus* in a sample which comprises using an antibody according to any one of aspects 16 to 19.
23. Kit for detecting and/or quantifying linear autoinducing peptides from *S*. *aureus* in a sample characterized in that it comprises at least one antibody specific for a linear peptide of sequence according to any one of aspects 16 or 17, or an antiserum comprising said antibody, or a conjugate according to any one of aspects 4 to 12, or any combination thereof.

The invention is described in detail by way of the following examples which are to be considered as merely illustrative and not limitative of the scope of the invention.

### MATERIALS AND METHODS

### 1. CHEMISTRY

The structures of the linear peptides and their corresponding linear haptens are shown in **Table 1.**

**Table 1. Chemical structures of the linear form AlPs (1-4) used for the calibration curves in ELISA, and their corresponding haptens used for bioconjugation with carriers.**

| **Lineal AIPs** | **Lineal NH₂-PEG(2) AIPs** |
|---|---|
| **AIP1o** | **NH₂-PEG(2)-CO-AIP1o** |
| 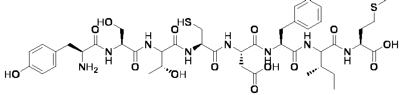 | 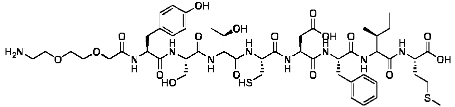 |
| **AIP2o** | **NH₂-PEG(2)-CO-AIP2o** |
| 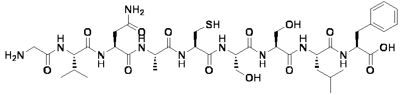 | 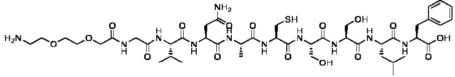 |
| **AIP3o** | **NH₂-PEG(2)-CO-AIP3o** |
| 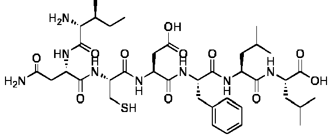 | 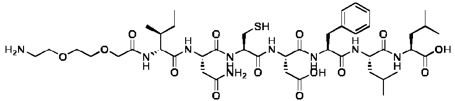 |
| **AIP4o** | **NH₂-PEG(2)-CO-AIP4o** |
| 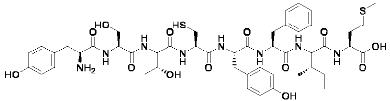 | 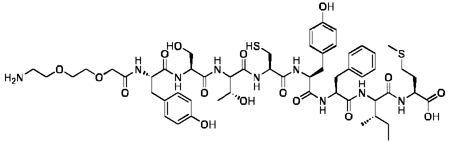 |

### General Procedures and Equipment

The reagents and solvents were purchased from commercial sources and were used without any further purification.

HPLC-PDA-MS analyses were performed on a Waters Alliance 2795 instrument with an automated injector and a Waters 2996 photodiode array detector coupled to an electrospray ion source (ESI-MS) Micromass ZQ mass detector and MassLynx 4.1 software. The instrument was operated in positive ESI (+) ion mode. All HPLC-PDA-MS analyses were performed using the elution conditions 1: XSelectTM C18 column (4.6 mm×50 mm, 3.5 µm). Elution solvent system: A: 0.1% HCOOH in H2O and B: 0.07% HCOOH in CH3CN. Gradient: 5% B to 100% B over 3.5 min at a flow rate of 1.6 mL/min. λ= 220 nm.

HPLC-PDA analyses were performed on a Waters Alliance 2695 with a Waters 2998 photodiode array detector using elution condition 2: XBridgeTM BEH130 C18 (3.5 µm, 4.5mm x 100mm) column. Elution solvent system: A: 0.045% TFA in H2O and B: 0.036% TFA in CH3CN. Flow rate 1.0 mL/min and temperature 25°C. λ =220nm. Gradient: 5-70%B in 8 minutes.

Purification was performed on Waters semi-preparative HPLC, separation module 2545, UV detector 2487 with dual absorbance. Column, XBridge BEH 130 semi-preparative C18 5 mm, 19x100 mm. Eluents, A (water with 0.1% TFA acid), B (ACN with 0.1% TFA acid), flow rate 16 mL/min. Detection wavelength 220 nm.

### 1.1. Preparation of lineal AIPs (AIPo 1-4) (analytes)

Linear AIPs 1-4 derivatives used for the preparation of immunoreagents and as controls were synthesized at the Peptide Synthesis Unit of NANBIOSIS ICTS (U3; https://www.nanbiosis.es/portfolio/u3-synthesis-of-peptides-unit/). Linear AIP peptides were manually synthesized on solid phase using 0.2 g of 2-chlorotrityl chloride resin (CTC resin; f= 1.6 mmol Cl/g). The resin was conditioned by washing with dichloromethane (DCM) and dimethylformamide (DMF). The C-terminal residue was introduced onto the solid support by addition of the corresponding Fmoc-AA-OH (0.6 mmol) and diisopropylethylamine (8.0 mmol) dissolved in anhydrous DCM. After 1.5 h, MeOH (0.8 mL/g de resina, 400 µL) was added to block the unreacted chloride groups and the mixture was allowed to react for 30 min. The mixture was then filtered and the resin washed with DMF and DCM. Peptide sequence elongation was performed by a series of Fmoc elimination and coupling cycles using a Fmoc/tBu protecting group strategy. All functional groups of the amino acid side chains were protected with tert-butyl ether (tBu), except for the cysteine thiol, which was protected with a trityl group (Trt). Fmoc elimination was performed by treatments (2 x 5 min) with piperidine-DMF solution (2:8, v/v). Amino acid couplings were performed by adding Fmoc-AA-OH (3 equiv.), N,N'-diisopropylcarbodiimide (DIC), 3 equiv. and 2-cyano-2-(hydroxyimino)acetic acid ethyl ester, potassium salt (K-Oxyme pure), 3 equiv. in 1.5 ML DMF for 45 min. After each Fmoc elimination or coupling step, the resin was washed with DMF, DCM. The linear AIPs 1-4 peptidyl-resins were cleaved using mild acidolysis with TFA-H₂O-TIS (95:2,5:2,5), for 1:30 hours. Then, the cleavage mixture was precipitated on cold diethyl ether, decanted, dissolved in H₂O-CH₃CN (1:1) and lyophilized to obtain AIPs 1-4 Linear which were purified by semi-preparative HPLC.

**Linear AIP1 peptide (AIP1o):** 95m g of linear AIP1 crude. Yield: 73.53%. Purified peptide: 25.8 mg. Purity determined by HPLC (220nm): 99.7% LR-MS: calculated mass for C₄₃H₆₂N₈O₁₄S₂: 979.13 [M+H]⁺, found by HPLC-MS (ESI): 979.2 [M+H]⁺.

**Linear AIP2 peptide (AIP2o):** 145mg of linear AIP2 crude. Yield: 79.71%. Purified peptide: 15.8 mg. Purity determined by HPLC (220 nm): 98.9%. LR-MS: calculated mass for C₃₈H₆₀N₁₀O₁₃S: 897.01 [M+H]⁺, found by HPLC-MS (ESI): 897.4 [M+H]⁺.

**Linear AIP3 peptide (AIP3o):** 205.1 mg linear AIP3 crude were obtained. Yield: 72. 06%. Purified peptide: 21.6 mg. Purity determined by HPLC (220 nm): 97.7 %. LR-MS: calculated mass for C₃₈H₆₀N₈O₁₁S: 837 [M+H]⁺, found by HPLC-MS (ESI): 837.5[M+H]⁺.

**Linear AIP4 peptide (AIP4o):** 96 mg of linear AIP4 crude. Yield: 77,92%. Purified peptide: 16.4 mg. Purity determined by HPLC (220 nm): 96.7%. LR-MS: calculated mass for C₄₈H₆₆N₈O₁₃S₂: 1027.21, found by HPLC-MS (ESI): 1027.3.

### 1.2. Preparation of NH₂-PEG(2)-CO-AIPo 1-4 (haptens)

The NH₂-PEG(2) AIPs 1-4 were synthesized in the same manner of AIPs 1-4 (analytes) except that Boc-PEG(2)-COOH was added to the N-terminal amino acid of each AIP 1-4 peptidyl resin. After Fmoc elimination, the linear NH2₂-PEG(2) AIPs 1-4 peptidyl-resin.

**Linear NH₂-PEG(2)-CO-AIP1o peptide (I):** 202 mg of linear NH₂-PEG(2)-AIP1o crude. Yield: 80.22%. Purified peptide: 51.7 mg. Purity determined by HPLC (220 nm): 98.6%. LR-MS: calculated mass for C₄₉H₇₃N₉O₁₇S₂:1124.28 [M+H]⁺, found by HPLC-MS (ESI): 1124.3 [M+H]⁺.

**Linear NH₂-PEG(2)-CO-AIP2o peptide (II):** 201.5 mg of linear NH₂-PEG(2)-AIP2o crude. Yield: 80.01%. Purified peptide: 29 mg. Purity determined by HPLC (220 nm): 98.6%. LR-MS: calculated mass for C₄₄H₇₁N₁₁O₁₆S:1042.17[M+H]⁺, found by HPLC-MS (ESI): 1042.3 [M+H]⁺.

**Linear NH₂-PEG(2)-CO-AIP3o peptide (III):** 202.3 g of linear NH₂PEG(2)-AIP3o crude. Yield: 80.8%. Purified peptide: 34 mg. Purity determined by HPLC (220 nm): 97.8%. LR-MS: calculated mass for C₄₄H₇₁N₉O₁₄S: 982.16 [M+H]⁺, found by HPLC-MS (ESI): 982.6 [M+H]⁺.

**Linear NH₂-PEG(2)-CO-AIP4o peptide (IV):** 166 mg of NH₂PEG(2)-CO-AIP4o crude. Yield: 88.53%. Purified peptide: 85.1 mg. Purity determined by HPLC (220 nm): 96.9%. LR-MS: calculated mass for C₅₄H₇₇N₉O₁₆S₂: 1172.37 [M+H]⁺, found by HPLC-MS (ESI): 1172.6 [M+H]⁺.

The sequences of these compounds are: I) NH₂-PEG(2)-CO-YSTCDFIM (SEQ ID NO:1)-OH, (II) NH₂-PEG(2)-CO-GVNACSSLF (SEQ ID NO: 2) -OH, (III) NH₂-PEG(2)-CO-INCDFLL (SEQ ID NO:3) -OH and (IV) NH₂-PEG(2)-CO-YSTCYFIM (SEQ ID NO:4) -OH.

### 2. IMMUNOCHEMISTRY

### General Procedures and Equipment

The reagents used were obtained from Aldrich Chemical Co. (Milwaukee, WI, USA) and from Sigma Chemical Co. (St. Louis, MO, USA). Purification of conjugates was carried out in ÄKTA Prime Plus using 2 HiTrap^{®} Desalting Columns both from Cytiva (Little Chalfont, Buckinghamshire, United Kingdom) and by dialysis using Spectra/Por^{®} membranes from Spectrumlabs (Piraeus, Greece, EU) molecular weight cut-off of 12-14 kDa. The matrix-assisted laser desorption ionization time-of-filght mass spectrometer (MALDI-TOF-MS) was a Bruker autoflex III Smartbeam spectrometer (Billerica, Massachusetts). The NanoDrop^{™} One is a UV-Visible spectrophotometer from Thermo Fisher Scientific Inc. (Madison, WI, USA). The pH and the conductivity of all buffers and solutions were measured with a SevenCompact^{™} Duo S213 pH-meter from Mettler-Toledo Gmbh (Greifensee, Switzerland). Microtiter pates (clear C-Shaped Immuno Nonsterile 96-Well Plates) were purchased from Nunc (Maxisorp, Roskilde, Denmark). Dilution pates (clear flat-shaped Nonsterile 96-Well plates) were purchased from Nirco (Barberà del Vallés, Spain). Washing steps were performed on a Biotek 405^{™} LS Microplate Washer (BioTek Inc., Vermont, USA). Absorbances were read on a Multiskan^{®} GO spectrophotometer (Thermo Fisher Scientific Oy, Vantaa, Finland) at a single wavelength mode (450 nm). The competitive curves were analysed with a four-parameter logistic equation using he software GraphPad Prism 8.0 (GraphPad Software Inc., San Diego, CA, USA). Clinical isolates were grown in polystyrene 96-well U-bottom with low evaporation lid (Corning Incorporated, NY, USA) inside the Varioskan^{™} LUX multimode microplate reader (Thermo Scientific, Vantaa, Finland).

### Buffers

Phosphate buffer saline (PBS) was 0.01 M phosphate buffer (1.48 mM KH2PO4 and 8.3 mM Na2HPO4) in a 0.8% saline solution (137 mmol/L NaCl, 2.7 mmol/L KCI), at pH 7.5. PBS with Tween20 (PBST) corresponds to 10 mM phosphate buffer the previous PBS solution with 0.05% Tween 20 (pH 7.5). Citrate buffer corresponds to 40 mM sodium citrate, pH 5.5. The substrate solution contains 0.375 µg mL⁻¹ of 3,3'5,5'-tetramethylbenzidine (TMB) and 0.004% H₂O₂ prepared in citrate buffer. Borate buffer corresponds to 0.2 M sodium borate/boric acid (pH 8.7). Coating buffer is a 0.05 M carbonate-bicarbonate buffer pH 9.6. All buffers were prepared using ultra-pure MilliQ^{®} water with a resistivity between 16-18 mΩ·cm.

### Analysis of Hapten Density

Hapten densities of BSA conjugates were estimated with MALI-TOF-MS comparing the molecular weight of carrier proteins with the molecular weight of the conjugates. MALDI plate was prepared by adding first 2 µL of the matrix solution (10 mg mL⁻¹ of α-Cyano-4-hydroxycinnamic acid in CH₃CN/H₂O 70:30, 0.1% HCOOH) until dry, followed by 2 µL of the purified sample diluted ½ with CH₃CN/H₂O (0.1% HCOOH) also letting it dry. Finally, 2 µL of the matrix solution are added over the mixture and left to dry. The resulting sample spots were ionized and detected by MALDI-TOF. Hapten densities were calculated through the equation: [MW(conjugate)-MW(BSA)]/[MW(hapten)-MW(lost atoms)].

### 2.1. IMMUNOGENS AND COATING ANTIGENS PREPARATION

### 2.1.1. Conjugation of I and III haptens with DMP as a cross-linker

The AIP1o and AIP3o haptens were successfully conjugated to the proteins BSA (Bovine Serum Albumin) and KLH (Keyhole Limpet Hemocyanin). For these means, DMP (dimethyl pimelimidate· 2HCl) was used as a cross-linker between hapten and protein. DMP contains an imidoester at each end of a 7-carbon spacer arm and forms an amidine bond with amino groups at alkaline pH.

Each hapten (3.4 mg AIP1o and 2.97 mg AIP3o) were dissolved in 600 µL CH₃CN/H₂O 1:1. A solution of 11.5 mg of BSAand another of 11.5 mg KLH were prepared in 2 mL borate buffer (pH 8.7). 2.353 mg DMP were dissolved in 1.5 mL borate buffer. Each hapten was added dropwise over BSA and KLH solutions while gentle stirring. Right after the DMP solution is added dropwise also while stirring and covered from light, due to the sensitivity of DMP to light. The reaction mixture was stirred for 4 hours at room temperature and kept overnight at 4°C.

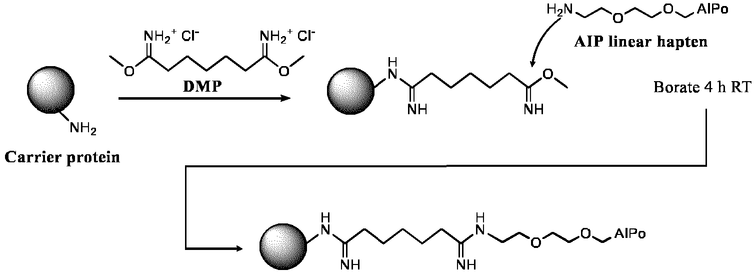

The following day, the bioconjugates were purified by dialysis using Spectra/Por membranes from Spectrumlabs (Piraeus, Greece, EU) with molecular weight cut-off of 12-14 kDa. The dialysis was started with 0.5 mM PBS (4 × 5 L) and ended up with ultrapure water (1 × 5 L).

The final samples were characterized by MALDI-TOF, rendering hapten densities of 8 and 4 haptens per molecule of BSA, for AIP1o and AIP3 conjugates, respectively (see **Table 2).** KLH conjugates cannot be analyzed by MALDI-TOF, therefore hapten density was evaluated by comparing with a parallel conjugation procedure with BSA conjugates. The samples were frozen at -80°C to and lyophilized (Telstar, LyoQuest) to be stored at -20°C. Working aliquots were prepared and stored at 4°C in 10 mM PBS at 1 mg mL⁻¹.

**Table 2. Hapten density of the conjugates (BSA=1) and immunogens (KLH=2) produced with the haptens with linear AIP1 and AIP3 structures linked through N-terminus (R1)**

| | **Hapten density** |
|---|---|
| **I-1** | 8 |
| **I-2** | - |
| **III-1** | 4 |
| **III-2** | - |

### 2.1.2. Conjugation of I, II, III and IV haptens with SIA as a cross-linker by EDAc method

The conjugation procedure was carried out in parallel in a solution of 50 mg BSA (Bovine Serum Albumin) and 40 mg of KLH (Keyhole Limpet Hemocyanin), for the conjugation of 4 conjugates, including a negative control conjugate with BSA. BSA was dissolved in 6.45 mL of Borax/Borate buffer pH=8.7, whereas the stock solution of KLH was diluted also in Borax/Borate buffer to reach the same concentration of the BSA solution (7.6 mg/mL).

N-hydroxysuccinimide iodoacetate (SIA) was prepared by dissolving first iodoacetic acid (783.8mg, 4 mmols) and NH2S (471.8mg, 4 mmols, 1eq) in 6mL of CH₂Cl₂ aNH2ydride and adding to this 845.95mg (4 mmols, 1eq) of DCC dissolved in 4mL CH₂Cl₂. The DCC was added to the flask in an ice-bath as the reaction is exothermic. The solution was left for 2 hours at 4°C followed by 1 hour at room temperature. The dicyclohexylurea is separated by filtration and the solution is evaporated. Warm ethyl acetate (AcOEt) was added and the reaction is left in an ice-bath for 30 minutes until the urea is seen precipitated, where it was then separated by filtration. The solution was evaporated once more and redissolved in warm CH₂Cl₂. The product was precipitated with EtO and cooled down at 4 °C. The product was obtained by filtration, washed with EtO and evaporated.

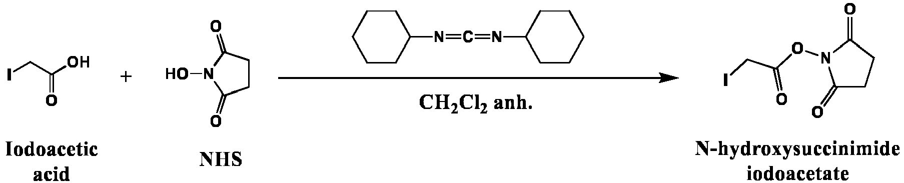

6 mg and 4.8 mg of succinimidyl iodoacetate (SIA) were dissolved separately in 500 µL of anhydrous DMF. SIA solution was added dropwise to the stirring solutions of BSA and KLH, respectively. The reactions were stirred during 4h at RT and left overnight at 4°C without agitation. The resulting solutions of BSA-iodoacetate and KLH-iodoacetate were purified by AKTA using 2 HiTrap^{®} Desalting Columns and eluting with Borax/Borate buffer. The protein concentration of both solutions was analysed by NanoDrop^{™} One spectrophotometer. Furthermore, the BSA-iodoacetate was analysed by MALDI-TOF-MS to determine the number of SIA molecules attached to each carrier protein.

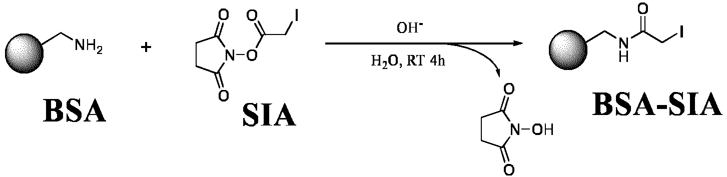

The iodoacetate groups linked to the carrier proteins were reacted with aminoacetaldehyde dimethyl acetal (2,2-dimethoxyethylamine) (C₄H₁₁NO₂) in a proportion of SIA:C₄H₁₁NO₂ 1:1.3 to ensure that all the iodine groups carry out a nucleophilic substitution S_{N}2 by the primary amino group of the C₄H₁₁NO₂. The required C₄H₁₁NO₂ quantities were dissolved in 50 µL of ultrapure H₂O and added dropwise to BSA-iodoacetate (BSA-SIA) and KLH-iodoacetate (KLH-SIA) solutions. The reaction was left stirring 3h at RT and analysed with MALDI-TOF-MS.

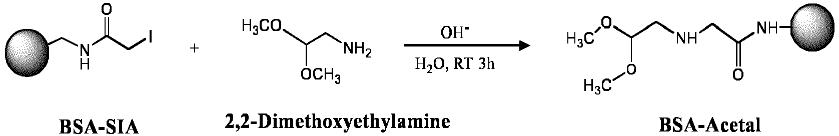

The BSA-acetal and KLH-acetal solutions are acidified until a pH=6 during 30 minutes by adding 3 drops of HCl 5M while stirring. The acidic hydrolysis causes the conversion of the acetal into a hemiacetal, and this into al aldehyde.

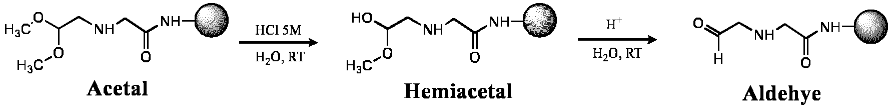

Once the solutions of BSA and KLH are functionalized with an aldehyde group, the solutions were distributed in 4 different vials each (5 in the case of BSA due to the control sample). The AIPo 1-4 haptens were dissolved in 600 µL acetonitrile:H₂O 1:1 and added dropwise in a proportion of SIA:Hapten 1:1. Straightaway, sodium cyanoborohydride (NaBH₃CN) dissolved in 100 µL Milli-Q water is added dropwise as a mild reducing agent in a proportion of 6 mol NaBH₃CN per 1 mol of hapten. **¡Error! No se encuentra el origen de la referencia.¡Error! No se encuentra el origen de la referencia.**

The final conjugate formula is the following:

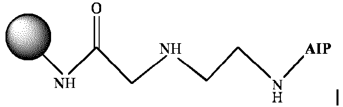

The final conjugates were purified by dialysis against 0.5 mM PBS (5 x 5 L) and Milli-Q water (1 x 5 L), and stored freeze dried at -80 °C. A small fraction (20 µL) of each conjugate was kept for MALDI-TOF analysis, rendering a hapten density of 4 haptens per molecule of BSA for the conjugates with haptens AIP1o, AIP2o, and AIP3o, and a hapten density of 5 for the conjugate with AIP4o. KLH conjugates cannot be analysed by MALDI-TOF, therefore hapten density was evaluated by comparing with a parallel conjugation procedure with BSA conjugates. Working aliquots were prepared and stored at 4 °C in 10 mM PBS at 1 mg mL⁻¹.

**Table 3** shows the conjugate density for the protein-SIA compounds after purification step.

**Table 3. Number of SIA residues linked to the carrier protein BSA.**

| | **Quantity (mg)** | **Yield (%)** | **N° of residues** |
|---|---|---|---|
| **BSA-SIA** | 40.05 | 80.1 | 12 |
| **KLH-SIA** | 29.4 | 73.5 | 12 |

**Table 4** shows the conjugate density of the intermediate and final conjugates:

**Table 4. Concentration of bioconjugates produced and hapten density of BSA cojugates calculated from MALDI-TOF analysis. Concentration was calculated by Bradford assay and confirmed by NanoDrop One^{™} spectrophotometer. KLH conjugates cannot be annalyzed by MALDI-TOF, therefore hapten density was evaluated by comparing with a parallel conjugation procedure with BSA conjugates.**

| | **Concentration (mg/mL)** | **Hapten density** |
|---|---|---|
| **BSA-SIA** | - | 12 |
| **BSA-SIA-Acetal** | - | 11 |
| **I-1** | 1.45 | 4 |
| **I-2** | 2.57 | - |
| **II-1** | 1.91 | 4 |
| **II-2** | 1.79 | - |
| **III-1** | 1.69 | 4 |
| **III-2** | 2.39 | - |
| **IV-1** | 2.64 | 5 |
| **IV-2** | 2.07 | - |

### 2.2. ANTIBODY PRODUCTION

### 2.2.1. Antibodies produced with immunogens containing DMP as a a cross-linker

As a first approach, polyclonal antibodies were obtained by immunizing 4 female New Zealand white rabbits with the immunogens I-2, and III-2 prepared with the DMP cross-linker. The protocol used for the production of antibodies was conducted in accordance with the institutional guidelines under a license from the local government and approved by the Institutional Animal Care and Use Committee at the CID-CSIC.

**Table 5. Antisera obtained following immunization with the linear form of AIP1 and AIP3 containing DMP as a cross-linker. For each case, the immunogen used for animal immunization and the coating antigen employed for ELISA development are indicated. Bioconjugates are denoted as follows: 1 = BSA, 2 = KLH.**

| **Immunogen** | **Antisera** | **Coating antigen** |
|---|---|---|
| **I-2** | As427, As428 | I-1 |
| **III-2** | As429, As430 | III-1 |

The antibody titer was assessed during the immunization process with non-competitive indirect ELISAs. Microtiter plates were coated with a fixed concentration of the homologous competitor conjugate (1 mg mL⁻¹) and the avidity of the production antibodies was measured by preparing serial dilutions of the corresponding As. The final blood serums of the animals were extracted after 5 immunizations, which was collected in vacutainer tubes provided with a serum separation gel. All tubes were centrifuged at 4°C for 10 min at 10 000 rpm to obtained the antisera, and stored at -80°C with 0.02% of sodium azide (NaN₃).

### 2.2.2. Antibodies produced with immunogens containing SIA as a cross-linker

### 2.2.2.1. Polyclonal antibody production

Polyclonal antibodies were obtained by immunizing 12 female New Zealand white rabbits with the corresponding immunogen prepared by the EDAc method, named I-2, II-2, III-2 and IV-2 (see **Table 6).**

**Table 6. Antisera obtained following immunization with the linear form of AIPs 1-4 containing SIA as a cross-linker. For each case, the immunogen used for animal immunization and the coating antigen employed for ELISA development are indicated. Bioconjugates are denoted as follows: 1 = BSA, 2 = KLH.**

| **Immunogen** | **Antisera** | **Coating antigen** |
|---|---|---|
| **I-2** | As431, As432, As433 | I-1 |
| **II-2** | As434, As435, As436 | II-1 |
| **III-2** | As437, As438, As439 | III-1 |
| **IV-2** | As440, As441, As442 | IV-1 |

The antibody titer was assessed during the immunization process with non-competitive indirect ELISAs. Microtiter plates were coated with a fixed concentration of the homologous competitor conjugate (1 mg mL⁻¹) and the avidity of the production antibodies was measured by preparing serial dilutions of the corresponding As. The final blood serums of the animals were extracted after 5 immunizations, which was collected in vacutainer tubes provided with a serum separation gel. All tubes were centrifuged at 4° for 10 min at 10 000 rpm to obtained the antisera, and stored at -80°C with 0.02% of sodium azide (NaN₃).

### 2.2.2.2. Monoclonal antibody production

With the aim to produce monoclonal antibodies (mAbs) against AIP1o, 4 female *Mus musculus* (BALB/c) of 6-8 weeks mice were immunized with I-2 (see **Table 7).** The protocol used for the production of antibodies was conducted in accordance with the institutional guidelines under a license from the local government and approved by the Institutional Animal Care and Use Committee at the CID-CSIC.

The antisera (As) obtained by immunizing the mice were named as:

**Table 7. Antisera obtained during the immunization procedure with linear form of AIP1 containing SIA as spacer arm for the production of monoclonal antibodies. The immunogen used was AIP1o-SIA-KLH (1-2). The antibody titer was assessed using AIP1o-SIA-BSA (I-1) in homologous format. Antisera were collected from all four mice, labelled as As M1-M4.**

| **Immunogen** | **Antisera** | **Coating antigen** |
|---|---|---|
| I-2 | As M1, As M2, As M3, As M4 | Homologous BSA conjugate (I-1) and heterologous BSA conjugate (closed form AIP1 or AIP1c) |

The antibody titer was assessed during the immunization process with non-competitive indirect ELISAs. Microtiter plates were coated with a fixed concentration of the homologous competitor conjugate (1 mg mL⁻¹) and the avidity of the production antibodies was measured by preparing serial dilutions of the corresponding As. The final blood serums of the animals were extracted by cardiac puncture after 6 immunizations. All blood samples were centrifuged at 4° for 10 min at 4000 rpm to obtained the antisera, and stored at -20°C with 10% of phosphate buffer saline (PBS) 10 mM.

After scarifying the mice with CO_{2,} the spleen is extracted and treated in order to obtain lymphocytes that will be used in the hybridoma technology to produce monoclonal antibodies against AlP1o. The total number of lymphocytes obtained was 59x10⁶ cells, which were fused with myeloma cells of P3X63Ag8.653 cell line (American Type Culture Collection ATCC). A total of 6 96-well polystyrene culture plates (flat-bottom) were seeded (100 mL/well).

After 10 days, the plates were examined to determine the fusion efficiency which was of 85,93%. Screening of hybridoma cells by ELISA and cloning cycles were performed to obtain a single hybridoma cell capable to produce monoclonal antibodies against AIP1o-SIA-KLH. Three final clones (C1.1.2.4.1.1, C1.1.2.4.1.2, C1.1.2.4.1.3) were selected.

### 2.3. ELISA PROCEDURE

Using specific antibodies developed against the linear forms, the quantity of linear AIPs can be quantified in two ways by using identical samples from the same bacterial culture:
- **Non-hydrolysed sample:** The untreated sample contains both cyclic and linear AIPs. The specific antibody detects **only the linear fraction (a).**
- **Hydrolysed sample (developed and optimised method):** The cyclic AIP fraction has been hydrolysed, converting all AIP molecules of the sample (potentially cyclic and open forms) into their linear form. The same specific antibody detects the **total quantity of AIP in the sample (b),** as all molecules are now in the linear form.

The amount of **cyclic AIP in the sample (c)** can be calculated by subtracting the fraction (a) from (b).

This immunoassay allows for the quantification of the cyclic fraction (c), linear fraction (a) and/or the total contribution of AIPs (b) in a sample, thereby providing novel clinical information (the actual quantity of AIPs, correlating with the amount of S. aureus). It was not considered previously using antibodies described in the state of the art. Furthermore, the detection of these forms of AIPs would represent a substantial improvement in the detection of S. aureus AIPs across various fields (biomedical research, clinical practice, food science, or environmental studies) compared to other technologies (e.g., HPLC-MS, as previously discussed), which have been scarcely reported for the detection of cyclic AlPs and have never achieved implementation in any of the aforementioned areas.

### 2.3.1.EXAMPLE 1 - AIP3o detection (As429FB, immunized with AIP3o-DMP-KLH)

### 2.3.1.1. Non-competitive indirect 2D ELISA for the detection of linear AIP3 (AIP3o)

The optimum concentrations of coating antigens and pAbs were evaluated by non-competitive indirect ELISAs. The coating antigens consists of both the homologous linear/opened competitor III-1 and the heterologous closed competitor (AIP3c-BSA). Initially, two-dimensional titration assays (2D-assay) were carried out assessing several dilutions of coating antigens in the plate (10 µg mL⁻¹ to 0.01 µg mL⁻¹, and zero in coating buffer; 100 µL well⁻¹) with serial dilutions of the pAbs obtained from the final blood (FB) sera, As428FB, As429FB and As430FB (by 1/2 dilutions starting at 1/500 to 1/32000 and zero in PBST). The experimental procedure of ELISA is fully detailed in the next section. The concentrations of BSA conjugate (coating antigen) and pAb's dilution chosen to perform competitive assays were selected at 70% of signal saturation that gives approximately 1 unit of absorbance (450 nm).

### 2.3.1.2. Competitive indirect ELISA As429/AIP3c-BSA for the detection of linear AIP3 (AIP3o)

A competitive assay was performed to detect AIP3o in a 96-well flat-bottom microplates, coated with 100 µL of BSA-AIP3c (heterologous closed form) coating buffer pH=9.6. The plate was incubated overnight at 4°C covered with an adhesive plate sealer to avoid contaminants.

AIP3 analyte was hydrolyzed with NaOH 0.1M during 10 min to open the thiolactone ring and obtain the linear forms of AIP 3. Serial dilutions were performed starting from 10 µM of analyte. 75 µL of each analyte dilutions and 75 µL of As429FB at 1/16000 were added to a dilution plate and incubated overnight at 4°C.

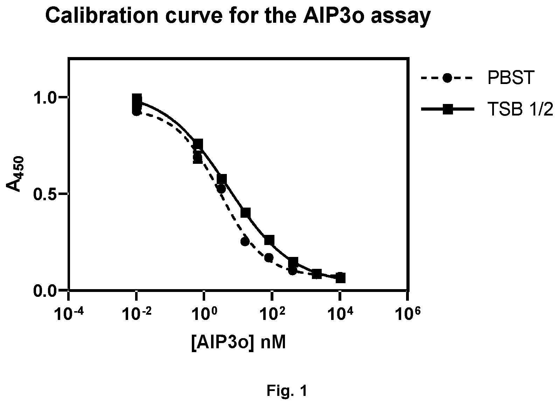

Next morning, after washing the coated plate with PBST (4x300 µL) with the Biotek 405^{™} LS Microplate Washer, 100 µL were passed per well from the dilution plate to the ELISA plate, and left without agitation 30 min at RT. After the competence time, a second washing step was performed, and a 1/6000 dilution of goat AntiRabbit IgG-HRP in PBST was added and incubated 30 min at RT. After the third and final washing, the next step consisted on adding 100 µL of the substrate solution (citrate solution, 0.0004% H₂O₂ and 0. 375 µg mL-1 TMB) and covered from light for 30 min. The colorimetric reaction was stopped by adding 50 µL of a H₂SO₄ 4M per well. The absorbance values were read at 450 nm and analyzed using GraphPad Prism 8.0 software. A standard curves was fitted to a four-parameter equation according to the formula y=B+(A-B)/[1-(x/C)^{D}], where A is the maximum absorbance, B is the minimum absorbance, C is the concentration at which a 50% of the absorbance is obtained, and D is the slope at the inflection point of the sigmoidal curve (see **Figure 1****).**

**Table 8. Parameters of competitive ELISA assay for the detection of Staphylococcus aureus QS molecule AIP3o. The data shown correspond to the average of 4 interday replicates with 3 well/replicates per concentration.**

| | **PBST 10 mM** | **TSB diluted ½ with PBST** |
|---|---|---|
| **As429FB dil.** | 1/16000 | 1/16000 |
| **III-1 (µg/mL)** | 0.062 | 0.080 |
| **Aₘᵢₙ** | 0.054 ± 0.03 | 0.087 ± 0.126 |
| **Aₘₐₓ** | 0.774 ± 9.191 | 0.999 ± 0.161 |
| **Slope** | -0.658 ± 0.131 | -0.463 ± 0.063 |
| **IC₅₀** | 7.576 ± 6.182 | 6,948 ± 3.487 |
| **LOD** | 0.213 ± 0.158 | 0.112 ± 0.043 |
| **R²** | 0.993 ± 0.003 | 0.997 ± 0.001 |

### 2.3.1.3. ELISA evaluation for the detection of AIP3o with the pAb As429FB Physicochemical parameters optimization

AIP3o assay performance was evaluated through the modification of different physicochemical parameters: analyte-antibody preincubation time, competence time, pH, conductivity (ionic strength) and presence of surfactant (% Tween 20).

**Table 9. Physicochemical parameters selected after AIP3o assay optimization. The parameters that improved the features of the assay were assessed separately and in conjunction.**

| | **Assay in PBST buffer** |
|---|---|
| **As dilution** | 1/16000 |
| **[Competitor]** | 0.1 µg/mL |
| **pH** | 7.5 |
| **Conductivity** | 15 mS/cm |
| **[Tween 20]** | 0.05 % |
| **Competition time** | 30 min |
| **Preincubation time Ab-AIP3o** | None |

### Specificity and Cross Reactivity studies

A calibration curve in the range of 10 mM to 0 nM in PBST buffer was prepared to use it in the optimized AIP3o ELISA with the corresponding pAb (As429FB) as described in section 2.3.1.2. The avidity of As429FB was assessed against all *S*. *aureus* QS molecules in the linear and closed forms (AIPo 1-4 and AIPc 1-4), and quinolone effector molecules of the pqs system from *P*. *aeruginosa,* as well as the QS molecules released by this pathogen, since it is also commonly found in infection samples. These analytes of *P*. *aeruginosa* evaluated in this study were Pyocianine (Pyo), 2-hydroxy-phenazine-1-carboxylic acid (OH-pnz), 2-(2-hydroxylphenyl)-thiazole-4-carbaldehyde) (IQS), 2-heptyl-3-hydroxyquinolone (PQS), 2-heptyl-4-quinolone (HHQ), 2-heptyl-4-hydroxyquinoline 1-oxide (HQNO), N-(3-oxo-dodecanoyl)-I-homoserine lactone (3-oxo-C12-HSL) and N-(3-oxododecanoyl)homoserine lactone (3-O-C₁₂-HSL).

Two replicate wells were used for each curve dilution and the average intensity of the two wells plotted. Taking as reference the calibration curve corresponding to the analyte initially selected as reference target, the cross-reactivity (CR) values were calculated according to the following equation: IC50 [nM] (Reference analyte)/ IC50 [nM] (cross-reactant) × 100.

**Table 10. Percentages of Cross Reactivity (CR) calculated for the assessed analytes using the pAb As429FB with the competitor AIP3c-AE-BSA.**

| Analyte | **IC₅₀ (nM)** | **% CR** |
|---|---|---|
| **AIP1c** | >10000 | < 0,05 |
| **AIP1o** | >10000 | < 0,05 |
| **AIP2c** | >10000 | < 0,05 |
| **AIP2o** | >10000 | < 0,05 |
| **AIP3c** | >10000 | < 0,05 |
| **AIP3o** | 4,709 | 100 |
| **AIP4c** | >10000 | < 0,05 |
| **AIP4o** | 1878 | < 0,05 |
| **Pyo** | >10000 | < 0,05 |
| **OH-pnz** | >10000 | < 0,05 |
| **IQS** | >10000 | < 0,05 |
| **PQS** | >10000 | < 0,05 |
| **HHQ** | >10000 | < 0,05 |
| **HQNO** | >10000 | < 0,05 |
| **3-oxo** | >10000 | < 0,05 |
| **HSL** | >10000 | < 0,05 |

### 2.3.1.4. Clinical Isolates with As429/AIP3c-BSA assay (AIP3o detection) Matrix effect study

Culture broth Tryptic Soy Broth (TSB) was diluted 1:2, 1:5, 1:10 and 1:20 and used to run the standard calibration curve of As429FB/AIP3c-AE-BSA assay. The TSB dilution that gave the best ELISA parameters was selected and the conditions of CA and As adjusted.

### Accuracy studies

Blind spiked samples using ½ diluted TSB culture broth were prepared, measured and interpolated in the standard curve mentioned above. The experiment was repeated three different days and the final accuracy results were expressed as mean of all replicates.

### Reproducibility study

The assay was performed three different days and in triplicates within the same day.

### Growth curves and AIP3o measurements

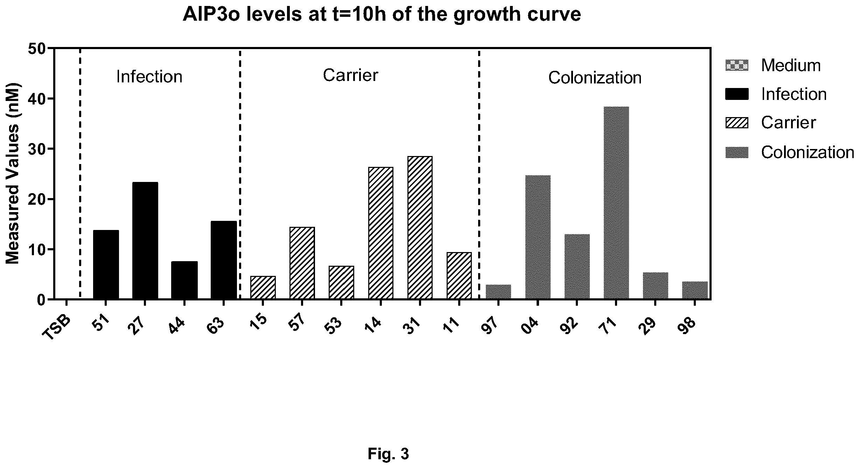

Clinical *S*. *aureus* isolates obtained from patients diagnosed with respiratory infections, colonization and carrier isolates and genotyped as *agr*-III were grown TSB culture broth plated in 96-well U-bottom culture plates for 10h at 37°C and 180 rpm shaking speed inside the Varioskan^{™} LUX microplate reader. The OD₆₀₀ values were measured every hour **(¡Error! No se encuentra el origen de la referencia.2)** and CFUs/mL were counted at time 10 h **(Table 11).** Then, aliquots were taken centrifuged and analysed using the developed immunochemical AIP3o assay. The results of the time point 10h are shown in **Figure 3****.**

**Table 11. Colony forming units (CFU/mL) of the bacterial isolates counted after 10 hours of growth inside the VarioskanTM LUX microplate reader, at 37°C and 180 rpm.**

| | **Colony Forming Units at 10h growth** | | |
|---|---|---|---|
| **Strain** | **Dilution** | **N° colonies** | **CFU/ml** |
| **20023351 (infection)** | **10⁻⁶** | **23** | **2,30E+09** |
| **20022115 (nasal carriage)** | **10⁻⁶** | **21** | **2,10E+09** |
| **20021357 (nasal carriage)** | **10⁻⁶** | **28** | **2,80E+09** |
| **20021753 (nasal carriage)** | **10⁻⁶** | **19** | **1,90E+09** |
| **30127314 (nasal carriage)** | **10⁻⁶** | **13** | **1,30E+09** |
| **1900631 (nasal carriage)** | **10⁻⁶** | **28** | **2,80E+09** |
| **20022611 (nasal carriage)** | **10⁻⁶** | **31** | **3,10E+09** |
| **20016297 (colonization)** | **10⁻⁶** | **12** | **1,20E+09** |
| **90690504 (colonization)** | **10⁻⁶** | **14** | **1,40E+09** |
| **90761892 (colonization)** | **10⁻⁶** | **24** | **2,40E+09** |
| **90871527 (infection)** | **10⁻⁶** | **10** | **1,00E+09** |
| **90110471 (colonization)** | **10⁻⁶** | **17** | **1,70E+09** |
| **90117244 (Infection)** | **10⁻⁶** | **13** | **1,30E+09** |
| **20022229 (colonization)** | **10⁻⁶** | **22** | **2,20E+09** |
| **90869563 (infection)** | **10⁻⁶** | **21** | **2,10E+09** |
| **90762598 (colonization)** | **10⁻⁶** | **19** | **1,90E+09** |

### 2.3.2.EXAMPLE 2 - AIP2o detection (As434FB, immunized with AIP2o-SIA-KLH)

### 2.3.2.1. Non-competitive indirect 2D ELISA for the detection of linear AIP2 (AIP2o)

The optimum concentrations of coating antigens and the pAbs were evaluated by non-competitive indirect ELISAs. The tested coated antigens consisted of both the homologous linear/opened competitor II-1 and the heterologous closed competitor (AIP2c-BSA).

A microtiter plate was coated with varying concentrations of the corresponding coating antigen, ensuring that each row contained a different concentration (ranging from 10 µg·m⁻¹ to 10 ng·mL⁻¹, and zero in coating buffer, 100 µL·well⁻¹). The plate was left overnight at 4°C. Subsequently, the plate was washed (4 x 300 µL, PBST), and different concentrations of antiserum (As434, As435, As436) were added (ranging from 1/500 to 1/32000, and zero in the assay buffer, 100 µL·well⁻¹. The plate was incubated for 30 minutes at room temperature, followed by a washing step (4 x 300 µL, PBST). Next, 100 µL/well of goat anti-rabbit IgG-HRP (1/6000 in PBST) was added, and the plate was incubated for an additional 30 minutes at RT. Subsequently, the plate was washed (4 x 300 µL, PBST), and the substrate solution (100 µL/well) was added, incubating another 30 minutes at room temperature, shielded from light. The enzymatic reaction was stopped by adding 4M H2SO4 (50 µL·well⁻¹), and the absorbance was finally read at 450 nm. The concentrations of BSA conjugate (coating antigen) and pAb's dilution chosen to perform competitive assays were selected at 70% of signal saturation that gives approximately 1 units of absorbance (450 nm).

### 2.3.2.2. Competitive indirect ELISA As434/AIP2c-BSA for the detection of linear AIP2 (AIP2o)

According to the results in Section 2.3.2.1, the heterologous competitor (AIP2c-BSA) was chosen due to its superior sensitivity to perform a competitive indirect ELISA. The competitive indirect assay was performed to detect AIP2o in a 96-well flat-bottom microplates, coated with 100 µL of AIP2c-BSA(heterologous) coating buffer pH=9.6. The plate was incubated overnight at 4°C covered with an adhesive plate sealer to avoid contaminants.

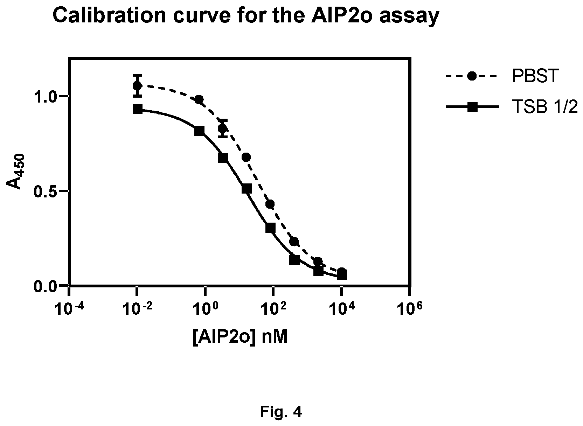

AIP2 analyte in the closed form was hydrolyzed with NaOH 0.1M during 10 min to open the thiolactone ring and obtain the linear forms of AIP2 (AIP2o). Serial dilutions were performed starting from 10 µM of analyte. 75 µL of each analyte dilutions and 75 µL of As434FB at 1/8000 were added to a dilution plate and incubated overnight at 4°C. Next morning, after washing the coated plate with PBST (4x300 µL) with the Biotek 405^{™} LS Microplate Washer, 100 µL were passed per well from the dilution plate to the ELISA plate, and left without agitation 30 min at RT. After the competence time, a second washing step was performed, and a 1/6000 dilution of goat AntiRabbit IgG-HRP in PBST was added and incubated 30 min at RT. After the third and final washing, the next step consisted on adding 100 µL of the substrate solution (citrate solution, 0.0004% H₂O₂ and 0. 375 µg mL-1 TMB) and covered from light for 30 min. The colorimetric reaction was stopped by adding 50 µL of a H₂SO₄ 4M per well. The absorbance values were read at 450 nm and analyzed using GraphPad Prism 8.0 software. A standard curves was fitted to a four-parameter equation according to the formula y=B+(A-B)/[1-(x/C)^{D}], where A is the maximum absorbance, B is the minimum absorbance, C is the concentration at which a 50% of the absorbance is obtained, and D is the slope at the inflection point of the sigmoidal curve (see **Figure 4****).**

**Table 12. Parameters of competitive ELISA assay for the detection of Staphylococcus aureus QS molecule AIP2o (As434/AIP2c-BSA ELISA system). The data shown correspond to the average of 4 interday replicates with 3 well/replicates per concentration.**

| | **PBST 10 mM** | **TSB diluted ½ with PBST** |
|---|---|---|
| **As434FB dil.** | 1/8000 | 1/8000 |
| **II-1 (µg/mL)** | 0.08 | 0.1 |
| **Aₘᵢₙ** | 0.057 ± 0.018 | 0.037 ± 0.009 |
| **Aₘₐₓ** | 1.105 ± 0.208 | 0.974 ± 0.088 |
| **Slope** | -0.670 ± 0.075 | -0.650 ± 0.112 |
| **IC₅₀** | 46.943 ± 8.152 | 9.135 ± 3.968 |
| **LOD** | 1.977 ± 0.002 | 0.329 ± 0.094 |
| **R²** | 0.996 ± 0.002 | 0.997 ± 0.003 |

### 2.3.2.3. ELISA evaluation for the detection of AIP2o with the pAb As434FB

### A. Physicochemical parameters optimization

AIP2o assay performance was evaluated through the modification of different physicochemical parameters: analyte-antibody preincubation time, competence time, pH, conductivity (ionic strength) and presence of surfactant (% Tween 20).

**Table 13. Physicochemical parameters selected after AIP2o assay optimization. The parameters that improved the features of the assay were assessed separately and in conjunction.**

| | **Assay in PBST buffer** |
|---|---|
| **As dilution** | 1/8000 |
| **[Competitor]** | 0.08 µg/mL |
| **pH** | 7.5 |
| **Conductivity** | 13.13 mS/cm |
| **[Tween 20]** | 0.05 % |
| **Competition time** | 30 min |
| **Preincubation time Ab-AIP2o** | Overnight (4°C) |

### B. Specificity and Cross Reactivity studies

A calibration curve in the range of 10 mM to 0 nM in PBST buffer was prepared to use it in the optimized AIP2o ELISA with the corresponding pAb (As434FB) as described in section 2.3.2.2. The avidity of As434FB was assessed against all *S*. *aureus* QS molecules in the linear and closed forms (AlPo 1-4 and AIPc 1-4).

Two replicate wells were used for each curve dilution and the average intensity of the two wells plotted. Taking as reference the calibration curve corresponding to the analyte initially selected as reference target, the cross-reactivity (CR) values were calculated according to the following equation: IC50 [nM] (Reference analyte) IC50 [nM] (cross-reactant) × 100.

**Table 14. Percentages of Cross Reactivity (CR) calculated for the assessed analytes using the pAb As434FB with the competitor AIP2c-AE-BSA.**

| **Analyte** | **IC₅₀ (nM)** | **% CR** |
|---|---|---|
| **AIP1c** | >10000 | < 0,05 |
| **AIP1o** | >10000 | < 0,05 |
| **AIP2c** | >10000 | < 0,05 |
| **AIP2o** | 48.65 | 100 |
| **AIP3c** | >10000 | < 0,05 |
| **AIP3o** | >10000 | < 0,05 |
| **AIP4c** | 2908 | 1.67 |
| **AIP4o** | >10000 | < 0,05 |

### 2.3.2.4. Clinical Isolates with As434/AIP2c-BSA assay (AIP2o detection)

### Matrix effect study

Culture broth Tryptic Soy Broth (TSB) was diluted 1:2, 1:5, 1:10 and 1:20 and used to run the standard calibration curve of As434FB/AIP2c-BSA assay. The TSB dilution that gave the best ELISA parameters was selected and the conditions of CA and As adjusted.

### Accuracy studies

Blind spiked samples using ½ diluted TSB culture broth were prepared, measured and interpolated in the standard curve mentioned above. The experiment was repeated three different days and the final accuracy results were expressed as mean of all replicates.

### Reproducibility study

The assay was performed three different days and in triplicates within the same day.

### Growth curves and AIP2o measurements

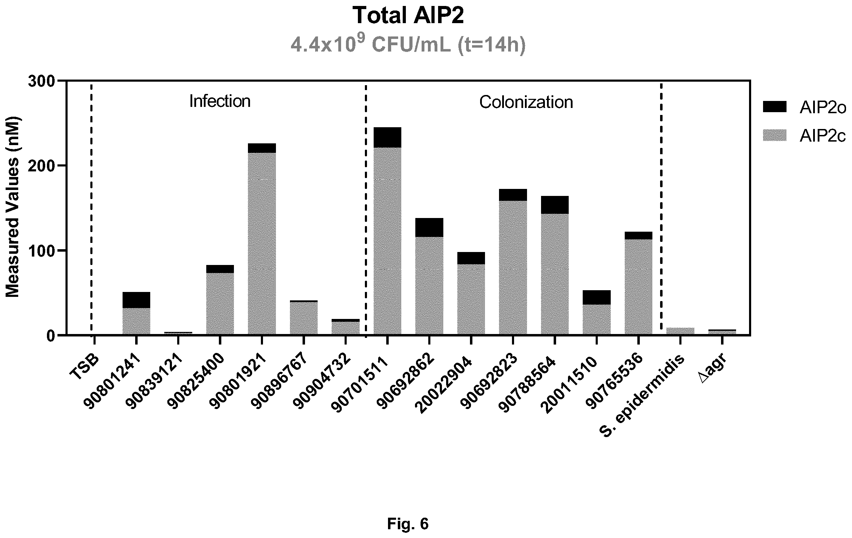

Clinical *S*. *aureus* strains obtained from patients diagnosed with respiratory infections, colonization and carrier isolates and genotyped as *agr-II* were grown in TSB culture broth cultures for 24h at 37°C and 180 rpm. The OD₆₀₀ values were measured at three different extraction times (9h, 14h, 24h) **(¡Error! No se encuentra el origen de la referencia.5)** and CFUs/mL were counted **(Table 15).** In addition, negative control strains S. *aureus* agr-negative (Δ*agr*) and *S. epidermidis* were also grown under the same conditions. Then, aliquots were taken centrifuged and analysed using the developed immunochemical AIP2o assay. The results of the time point 14h are shown in **Figure 6****.**

**Table 15. Colony forming units (CFU/mL) of the bacterial isolates counted at 0, 14 and 24 hours of growth at 37°C and 180 rpm shaking speed.**

| **Colony Forming Units at 14h growth** | | | |
|---|---|---|---|
| **Strain** | **Dilution** | **N° colonies** | **CFU/ml** |
| **90701511 (colonization)** | 10⁻⁶ | 20 | **2.00E+09** |
| **90692862 (colonization)** | 10⁻⁶ | 38 | **3.80E+09** |
| **90766623 (colonization)** | 10⁻⁶ | 15 | **1.50E+09** |
| **20022904 (colonization)** | 10⁻⁶ | 11 | **1.10E+09** |
| **90788564 (colonization)** | 10⁻⁶ | 21 | **1.50E+09** |
| **20011510 (colonization)** | 10⁻⁶ | 17 | **1.70E+09** |
| **90765536 (colonization)** | 10⁻⁶ | 23 | **2.30E+09** |
| **90801921 (infection)** | 10⁻⁶ | 19 | **1.60E+09** |
| **90896767 (Infection)** | 10⁻⁶ | 15 | **1.20E+09** |
| **90904732 (infection)** | 10⁻⁶ | 16 | **1.30E+09** |
| **90801241 (infection)** | 10⁻⁶ | 10 | **1.50E+09** |
| **90839121 (infection)** | 10⁻⁶ | 11 | **1.90E+09** |
| **90825400 (infection)** | 10⁻⁶ | 12 | **1.20E+09** |
| ***S. aureus* Δ*agr*** | 10⁻⁵ | 44 | **4.40E+08** |
| ***S. epidermidis*** | 10⁻⁵ | 50 | **5.00E+08** |

**Figure 6** shows the total amount of AIP2 in the supernatants extracted from clinical *Staphylococcus* aureus isolates and the relative contribution of the closed AIP2 (AIP2c) and linear AIP2 (AIP2o), as well as negative control strains *S. epidermidis* and agr-negative *S*. *aureus.* For the quantification of the total AIP2 amount, samples were subjected to the hydrolysis treatment as previously described in **Section 2.3.**

### BIBLIOGRAPHY:

1. Lowy, F. D., Staphylococcus aureus infections. N Engl J Med 1998, 339 (8), 520-32.
2. Schöfer, H.; Bruns, R.; Effendy, I.; Hartmann, M.; Jappe, U.; Plettenberg, A.; Reimann, H.; Seifert, H.; Shah, P.; Sunderkötter, C.; Weberschock, T.; Wichelhaus, T. A.; Nast, A., Diagnosis and treatment of Staphylococcus aureus infections of the skin and mucous membranes. J Dtsch Dermatol Ges 2011, 9 (11), 953-67.
3. Holland, T. L.; Baddour, L. M.; Bayer, A. S.; Hoen, B.; Miro, J. M.; Fowler, V. G., Jr., Infective endocarditis. Nat Rev Dis Primers 2016, 2, 16059.
4. Miro, J. M.; Anguera, I.; Cabell, C. H.; Chen, A. Y; Stafford, J. A.; Corey, G. R.; Olaison, L.; Eykyn, S.; Hoen, B.; Abrutyn, E.; Raoult, D.; Bayer, A.; Fowler, V. G., Jr., Staphylococcus aureus native valve infective endocarditis: report of 566 episodes from the International Collaboration on Endocarditis Merged Database. Clin Infect Dis 2005, 41 (4), 507-14.
5. Melzer, M.; Welch, C., Thirty-day mortality in UK patients with community-onset and hospital-acquired meticillin-susceptible Staphylococcus aureus bacteraemia. J Hosp Infect 2013, 84 (2), 143-50.
6. Shah, A. S. V.; McAllister, D. A.; Gallacher, P.; Astengo, F.; Rodriguez Pérez, J. A.; Hall, J.; Lee, K. K.; Bing, R.; Anand, A.; Nathwani, D.; Mills, N. L.; Newby, D. E.; Marwick, C.; Cruden, N. L., Incidence, Microbiology, and Outcomes in Patients Hospitalized With Infective Endocarditis. Circulation 2020, 141 (25), 2067-2077.
7. Lee, A. S.; de Lencastre, H.; Garau, J.; Kluytmans, J.; Malhotra-Kumar, S.; Peschel, A.; Harbarth, S., Methicillin-resistant Staphylococcus aureus. Nat Rev Dis Primers 2018, 4, 18033.
8. Rasigade, J. P.; Vandenesch, F., Staphylococcus aureus: a pathogen with still unresolved issues. Infect Genet Evol 2014, 21, 510-4.
9. David, M. Z.; Daum, R. S., Community-associated methicillin-resistant Staphylococcus aureus: epidemiology and clinical consequences of an emerging epidemic. Clin Microbiol Rev 2010, 23 (3), 616-87.
10. Jensen, S. O.; Lyon, B. R., Genetics of antimicrobial resistance in Staphylococcus aureus. Future Microbiol 2009, 4 (5), 565-82.
11. Chambers, H. F.; Deleo, F. R., Waves of resistance: Staphylococcus aureus in the antibiotic era. Nat Rev Microbiol 2009, 7 (9), 629-41.
12. WHO The top 10 causes of death.
13. Tal-Gan, Y; Ivancic, M.; Cornilescu, G.; Cornilescu, C. C.; Blackwell, H. E., Structural characterization of native autoinducing peptides and abiotic analogues reveals key features essential for activation and inhibition of an AgrC quorum sensing receptor in Staphylococcus aureus. J Am Chem Soc 2013, 135 (49), 18436-44.
14. Hermanson, G. T., Bioconjugate Techniques (2nd Edition). Elsevier: 2008.
15. Lipman, N. S.; Jackson, L. R.; Trudel, L. J.; Weis-Garcia, F., Monoclonal versus polyclonal antibodies: distinguishing characteristics, applications, and information resources. liar j 2005, 46 (3), 258-68.
16. Mitra, S.; Tomar, P. C., Hybridoma technology; advancements, clinical significance, and future aspects. J Genet Eng Biotechnol 2021, 19 (1), 159.

## Claims

1. Hapten of formula (A) 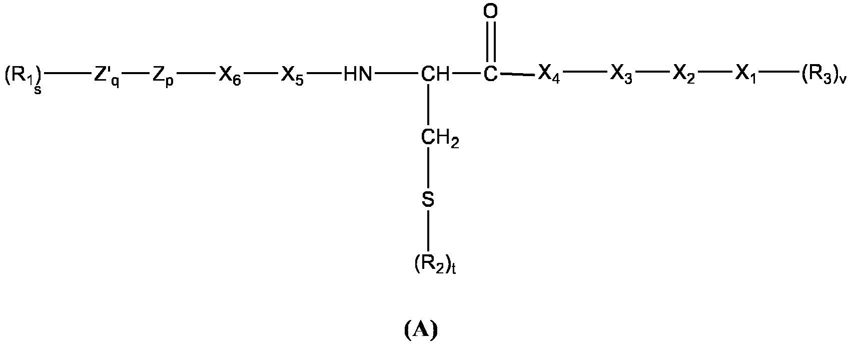 wherein
X₁ is selected from the group consisting of Methionine (M), Phenylalanine (F) and Leucine (L); X₂ is selected from the group consisting of Isoleucine (I) and Leucine (L); X₃ is selected from the group consisting of Phenylalanine (F) and Serine (S); X₄ is selected from the group consisting of Aspartic acid (D), Serine (S) and Tyrosine (Y); X₅ is selected from the group consisting of Threonine (T), Alanine (A) and Asparagine (N); X₆ is selected from the group consisting of Serine (S), Asparagine (N) and Isoleucine (I);
Z is selected from the group consisting of Tyrosine (Y) and Valine (V) and Z' is Glycine (G);
p, q are, independently, selected between 0 and 1
R₁, R₂ and R₃ are, independently, a spacer arm selected from the group consisting of R₄ and COR₄;
R₄ is selected from the group consisting of -(CH₂CH₂O)ₙ-, (C2-C10) alkyl, (C3-C10) alkenyl, (C3-C10) alkynyl and -(CH₂)m-aryl-(CH₂)r, substituted by -SH, NH₂, -OH, - COOH, -CHO, -CΞCH, halogen or -N₃; where n is an integer number selected between 2 and 5, m and r are selected, independently, between 0 and 3;
s, t and v are independently selected between 0 and 1; where if s is 1, t and v are 0; if t is 1, s and v are 0; and if v is 1, s and t are 0.

2. The hapten according to claim 1 where the spacer arm is -CO-(CH₂CH₂O)₂-NH₂.

3. The hapten according to any one of claims 1 or 2 selected from the group consisting of 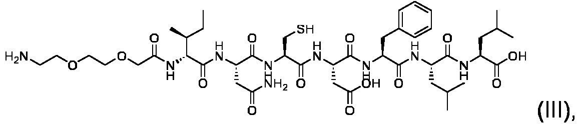 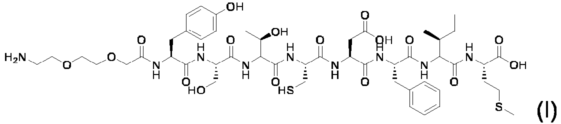 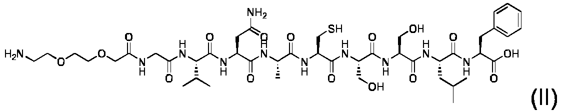 and

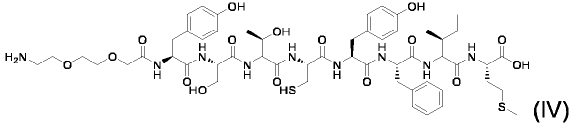

4. A conjugate comprising at least a hapten according to any one of claims 1 to 3 in combination with a second component selected from the group consisting of an antigenicity carrier protein or a fragment thereof, a detectable labelling agent, an oligonucleotide, a polysaccharide, a polymer and a support.

5. The conjugate according to claim 4, wherein the second component is an antigenicity carrier protein or a fragment thereof, preferably the carrier protein is selected from the group consisting of bovine serum albumin (BSA), horseshoe crab hemocyanin (HCH), keyhole limpet hemocyanin (KLH), rabbit serum albumin (RSA), horseradish peroxidase (HRP), ovoalbumin (OVA) conalbumin (CONA) thyroglobulin and fibrinogen, more preferably the carrier protein is BSA or KLH.

6. The conjugate according to any one of claims 4 to 5, wherein the hapten is bound covalently to the second component through a cross-linker, preferably the cross-linker is DMP (dimethyl pimelimidate) or SIA (N-hydroxysuccinimide iodoacetate).

7. The conjugate according to any one of claims 4 to 6, wherein the hapten is bound to the second component through the R1 position.

8. The conjugate according to any one of claims 4 to 7 wherein the hapten is selected from the group consisting of 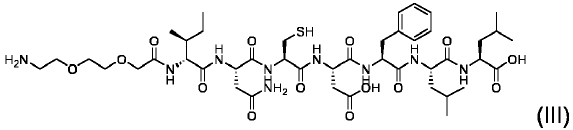 and

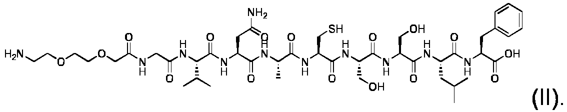

9. Use of the conjugate according to any one of claims 4 to 8 for obtaining an antibody or antisera.

10. Use according to claim 9, wherein the conjugate comprises a hapten selected from the group consisting of 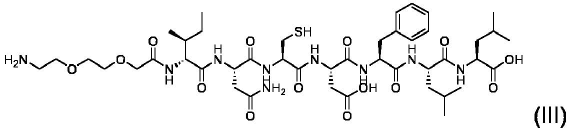 and

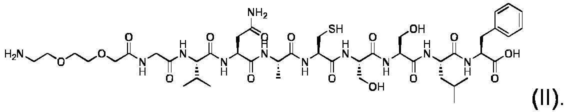

11. Antibody or antisera specific for a linear peptide selected from the group consisting of SEQ ID NO: 1, 2, 3 and 4.

12. Antibody or antisera according to claim 11 specific for a linear peptide of sequence SEQ ID NO: 3 or SEQ ID NO: 2.

13. Antibody or antisera according to any one of claims 11 to 12 obtained by immunizing a non-human mammal with a conjugate according to any one of claims 4 to 8, particularly wherein the carrier protein is KLH.

14. An in vitro method for detecting and/or quantifying linear autoinducing peptides from *S*. *aureus* in a sample which comprises the use of an antibody or antiserum as defined in claims 11 to 13, preferably wherein the detection and/or quantification is carried out by an immunochemical technique, preferably the immunochemical technique is an ELISA.

15. An *in vitro* method for diagnosing a *S*. *aureus* infection which comprises hydrolyzing a sample obtained from a patient in suitable conditions, and determining the amount of linear autoinducing peptides using an antibody according to any one of claims 11 to 13 or an *in vitro* method for the identification of *S*. *aureus* in a sample which comprises using an antibody according to any one of claims 11 to 13.
